(19) Europäisches Patentamt
European Patent Office
Office européen des brevets

(11) Publication number: **0 522 547 A2**

# (12) EUROPEAN PATENT APPLICATION

(21) Application number: **92111628.1**

(22) Date of filing: **09.07.92**

(51) Int. Cl.[5]: **C07C 237/12**, C22B 3/28, C22B 3/32, C11D 9/30, C01B 17/16, C01B 21/20

(30) Priority: **12.07.91 US 729514**

(43) Date of publication of application: **13.01.93 Bulletin 93/02**

(84) Designated Contracting States: **AT BE CH DE DK ES FR GB GR IT LI LU MC NL PT SE**

(71) Applicant: **THE DOW CHEMICAL COMPANY**
**2030 Dow Center, Abbott Road**
**Midland, MI 48640(US)**

(72) Inventor: **Garlich, Joseph R.**
**301 Southern Oaks Drive**
**Lake Jackson, Texas 77566(US)**
Inventor: **Simon, Jaime**
**Rt. 1, Box 120-G**
**Angleton, Texas 77515(US)**
Inventor: **Huff, Harrell L.**
**515 Oak Drive**
**Lake Jackson, Texas 77566(US)**
Inventor: **Crump, Druce K.**
**142 Oyster Creek Drive, Number 48**
**Lake Jackson, Texas 77566(US)**

(74) Representative: **Huber, Bernhard, Dipl.-Chem. et al**
**Patentanwälte H. Weickmann, Dr. K. Fincke F.A. Weickmann, B. Huber Dr. H. Liska, Dr. J. Prechtel, Dr. B. Böhm, Kopernikusstrasse 9, Postfach 86 08 20**
**W-8000 München 86(DE)**

## (54) Carbonyl-containing degradable chelants, uses and compositions thereof.

(57) New compounds are represented by Formulas 1 and 2 where Formula 1 is:

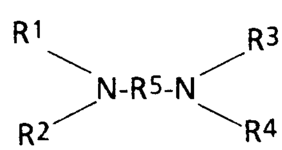

wherein each of $R^1$, $R^2$, $R^3$, and $R^4$ is independently H, hydroxyalkyl, $-C(R^6)_2COOH$ or ammonium, amine, or alkali metal salts thereof; $R^5$ is an alkylene group having at least one carbonyl group; and each $R^6$ is independently selected from H, and alkyl groups of from 1 to 4 carbon atoms, straight or branched chain, preferably H, $-CH_3$, or $-C_2H_5$, more preferably each $R^6$ is H and Formula 2 is:

EP 0 522 547 A2

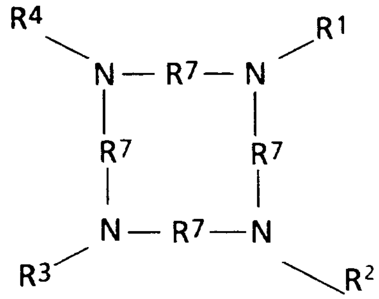

wherein $R^1$, $R^2$, $R^3$, $R^4$, $R^5$ and $R^6$ are defined for Formula 1, preferably at least 3, most preferably 4 of $R^1$, $R^2$, $R^3$, and $R^4$ are independently $-C(R^6)_2COOH$ or salts thereof; and each $R^7$ is independently $R^5$ or an alkylene group. The compounds are advantageously photo- and bio-degradable and are particularly useful as chelants especially in washing compositions and in gas conditioning.

Chelants or chelating agents are compounds which form coordinate covalent bonds with a metal ion to form chelates. Chelates are coordination compounds in which a central metal atom is bonded to two or more other atoms in at least one other molecule or ion (called ligand) such that at least one heterocyclic ring is formed with the metal atom as part or each ring.

Chelants are used in a variety of applications including food processing, soaps, detergents, cleaning products, personal care products, pharmaceuticals, pulp and paper processing, water treatment, metal working and metal plating solutions, textile processing solutions, fertilizers, animal feeds, herbicides, rubber and polymer chemistry, photofinishing, and oil field chemistry. Some of these activities result in chelants entering the environment. For instance, agricultural uses or detergent uses may result in measurable quantities of the chelants being in water. It is, therefore, desirable that chelants degrade after use.

Biodegradability, that is susceptibility to degradation by microbes, is particularly useful because the microbes are generally naturally present in environments into which the chelants may be introduced. Commonly used chelants like EDTA (ethylenediamine tetraacetic acid) are biodegradable, but at rates somewhat slower and under conditions considered by some to be less than optimum. (See, Tiedje, "Microbial Degradation of Ethylenediaminetetraacetate in Soils and Sediments," Applied Microbiology, Aug. 1975, pp. 327-329.) It would be desirable to have a chelating agent which degrades faster than EDTA or other commonly used chelants.

Photodegradation is also useful since light is frequently present in environments from which it is desired to remove chelants. Commonly used chelants like EDTA are known to undergo photodegradation, but again, at rates more slowly than would be desired for rapid removal from an environment. (See, Lochhart, et al. "Aerobic Photodegradation of Fe(III)(Ethylenedinitrilo)tetraacetate (Ferric EDTA); Implications for Natural Waters," Environmental Science & Technology, Vol. 9, No. 12, 1975, pp. 1035-1038.) It would be desirable to have a chelating agent which degrades more rapidly in the presence of light than does EDTA or other commonly used cheating agents.

While degradation of the chelant compounds themselves is an important factor in ascertaining their fate in the environment, it is also important to consider the form(s) in which the compound is likely to be found in a natural environment like a lake, river or soil. In contact with such environments, chelants can frequently be expected to be in the form of their chelates with metals present in the environment or metals acquired in use of the chelant. The specific metal chelated depends on the metals present, their relative concentrations and availability, and the relative affinity (for example as expressed by stability constants) of the chelant for each metal present. It is often important that the chelant degrade well in the form of its iron, copper, manganese or calcium complexes. It would be desirable for a chelant compound to degrade in the form(s) it is most likely to be found in the environment. This form is commonly the iron complex. (See, Laurent et al., IVL Report, "Effect of Complex Formers on the Aquatic Environment, NTA, EDTA and DTPA", Inst. Water and Air Conservation Research (IVL), Stockholm, Pub. B921, Dec. 1988.

Some chelants are at least somewhat biodegradable, but have other disadvantages that reduce their suitability for applications that may result in their presence in water.

In one aspect the invention is a compound represented by Formula 1:

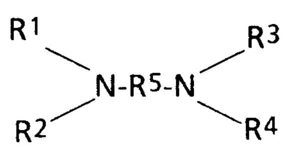

wherein each of $R^1$, $R^2$, $R^3$, and $R^4$ is independently H, hydroxyalkyl-$C(R^6)_2COOH$, or ammonium, amine, or alkali metal salts thereof, but at least 3 of $R^1$, $R^2$, $R^3$, and $R^4$ are -$C(R^6)_2COOH$ or salts thereof; $R^5$ is an alkylene group having at least one carbonyl group; and each $R^6$ is independently selected from H, and alky groups of from 1 to 4 carbon atoms, straight or branched chain, preferably H, -$CH_3$, or -$C_2H_5$, more preferably each $R^6$ is H. and Formula 2 is:

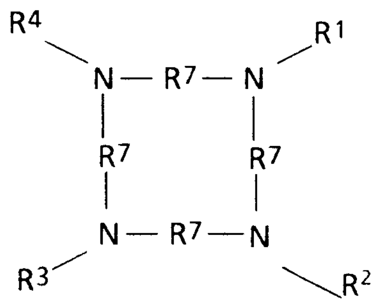

wherein $R^1$, $R^2$, $R^3$, $R^4$, and $R^5$ are defined for Formula 1, preferably at least 3, most preferably 4 of $R^1$, $R^2$, $R^3$, and $R^4$ are independently $-C(R^6)_2COOH$ or salts thereof; and each $R^7$ is independently $R^5$ or an alkylene group, but at least one $R^7$ is $R^5$.

In another aspect the invention is the method of using compounds of Formula 1 or 2 to chelate metal ions.

In another aspect, the invention is a washing composition comprising an organic detergent surfactant selected from the group consisting of anionic detergents, cationic detergent, nonionic detergents, ampholytic detergents, zwitterionic detergents, and mixtures of such detergents suitable for use in water and at least one water-soluble salt of the acids of Formula 1 or 2 selected from the group consisting of alkali metal salts, ammonium salts, and alkyl ammonium salts.

In another aspect, the invention is an aqueous washing system comprising an organic detergent surfactant selected from the group consisting of anionic detergents, cationic detergents, nonionic detergents, ampholytic detergents, zwitterionic detergents, and mixtures of the above and at least one water-soluble salt of an acid of Formula 1 or 2 selected from the group consisting of alkali metal salts, ammonium salts, and alkyl ammonium salts, the ratio by weight of the detergent surfactant to the salt being in the range of from 1:10 to 3:1, said system having a pH between 8 and 12.

In the field of gas conditioning, the invention includes a fluid comprising contacting the fluid with an aqueous solution of at least one lower valence state polyvalent metal chelate of Formula 1 or 2. Additionally the invention includes a fluid comprising contacting said fluid with an aqueous solution at a pH suitable for removing $H_2S$ wherein said solution contains at least one higher valence polyvalent metal chelate of Formula 1 or 2.

Compounds of the invention are effective chelants, and are advantageously especially useful in maintaining the bleaching effect of detergents.

The invention includes compounds of Formula 1

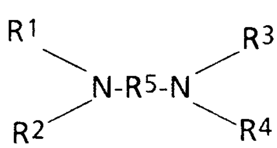

wherein each of $R^1$, $R^2$, $R^3$, and $R^4$ is independently H, hydroxyalkyl, $-C(R^6)_2COOH$, or ammonium, amine, or alkali metal salts thereof, but at least 2, preferably at least 3, most preferably all 4 of $R^1$, $R^2$, $R^3$, and $R^4$ are $-C(R^6)_2COOH$ or salts thereof because increasing the number of acid groups increases the denticity of a compound; $R^5$ is an alkylene group, preferably a straight chain alkylene group, preferably of from 2 to 3 carbon atoms and having at least one, preferably only one carbonyl group attached thereto to enhance biodegradation or photodegradation; and $R^6$ is independently selected from H, and alky groups of from 1 to 4 carbon atoms (straight or branched chain), preferably H, $-CH_3$, or $-C_2H_5$, more preferably each $R^6$ is H. The carbonyl group(s) suitably include any carbon atom(s) of $R^5$, preferably, for reasons of degradability at least one carbon atom to which an imino nitrogen is attached, more preferably each carbon adjacent to a carbonyl group has no functional group thereon (is most preferably a $-CH_2$-group). When $R^1$, $R^2$, $R^3$ or $R^4$ is a hydroxyalkyl group, preferably only one of the groups is hydroxyalkyl, and the hydroxyalkyl group preferably has from 1 to 5, more preferably from 2 to 4 carbon atoms, most preferably 3 carbon atoms.

The most preferred compound is that of Formula 1 wherein $R^1$, $R^2$, $R^3$ and $R^4$ are each $-CH_2COOH$, and $R^5$ is

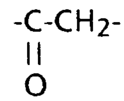

This compound is referred to herein as OXOEDTA.

Preferred compounds of Formula 1 include N,N'-(1-oxo-1,2-ethanediyl)bis[(N-(carboxymethyl)glycine]-(referred to herein as OXOEDTA); N,N'-[1-methyl-2-oxo-1,2-ethanediyl)]bis[N-(carboxymethyl)glycine]; N,N'-[1-ethyl-2-oxo-1,2-ethanediyl]bis[N-(carboxymethyl)glycine];N,N'-[1-oxo-1,2-ethanediyl]bis(N-(1-carboxyethyl)alanine]; N,N'-[1-methyl-2-oxo-1,2-ethanediyl]bis[N-(1-carboxyethyl)alanine]; N,N'-[1-ethyl-2-oxo-1,2-ethanediyl]bis[N-(1-carboxyethyl)alanine]; N,N'-[2-oxo-1,3-propanediyl]bis[N-(carboxymethyl)-glycine]; N,N'-[1-oxo-1,3-propanediyl]bis[N-(carboxymethyl)glycine]; N,N'-[1-oxo-1,3-propanediyl]bis[N-(1-carboxyethyl)alanine] and N,N'-[2-oxo-1,3-propanediyl]bis[N-(1-carboxyethyl)alanine], with OXOEDTA most preferred.

Compounds of Formula 1 are prepared by methods illustrated in the examples of the invention but with substitution of reactants having the desired carbon chain structures and substituents. Alternatively, the method of V. Cocheci, E. Gerasimou, C Csunderlik, L. Cotarca and A. Novac in Rev. Roum. Chim., 34(3), page 749-757, 1989 using ozone with tertiary amines to form disubstituted amides can suitably be used to make structures of Formula 1 from the corresponding tetra substituted diamines. For instance, EDTA would react with ozone to give N,N'-(1-oxo-1,2-ethanediyl)bis[N-(carboxymethyl)glycine].

Alternatively, the method of E.A.H. Roberts in Chemistry and Industry (London) dated July 29, 1961 page 1170-1171 using manganese dioxide to convert tertiary amines to disubstuted amides can be used to add carbonyl functionality to the amines. For instance, OXOEDTA can be prepared from EDTA by exposing EDTA to manganese dioxide by the disclosed methods.

Methods of making amides from amines and carboxylic anhydrides are disclosed in S. Patai "The Chemistry of Amides", 1970, p 86-91 (Interscience Publishers) and references therein. Thus, for instance iminodiacetic acid could react with nitrilotriacetic acid anhydride to make OXOEDTA by methods disclosed therein.

Alternatively, a method of producing substituted alpha-amino acids by reacting glyoxal with excess amine described by J. A. Field in Chemistry and Industry volume 60, page 960-963, 1947 is useful in preparing compounds of the invention by reacting such compounds as secondary amines with glyoxal in aqueous solutions to produce the amide of $\alpha$-aminoacetic acid. This chemistry has been further developed by P. Ferruti et al. in J. Chem. Soc. (C) page 2512-2513, 1970 and 2984-2985, 1971 and by J.M. Kleigman and R.K. Barnes in J.Heterocycl. Chem. page 1153-1155, 1970, where the reaction of glyoxal with disubstituted amines to give tetra substituted ethane products is described. The latter improvement is useful to produce compounds of the invention by reaction of the tetrasubstituted ethane with water to give fully substituted aminoacetamides an example of which is OXOEDTA. For example, reaction of glyoxal monohydrate with a secondary amine such as iminodiacetic acid would produce 1,1,2,2-tetra(iminodiacetic acid)-ethane. Reaction of this tetrasubstituted ethane with water would result in OXOEDTA.

Variations on these methods are within the skill in the art.

Additionally the invention includes compounds of Formula 2:

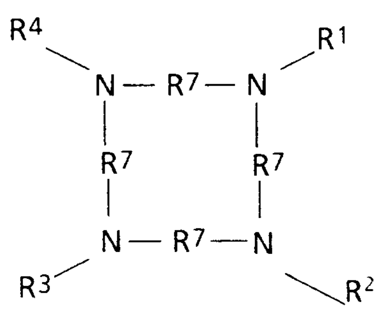

wherein $R^1$, $R^2$, $R^3$, $R^4$, $R^5$ and $R^6$ are defined as for Formula 1, but preferably at least 3, most preferably 4 of $R^1$, $R^2$, $R^3$, and $R^4$ are $-C(R^6)_2COOH$ or salts thereof (amine, ammonium, or alkali metal); and each $R^7$ is independently $R^5$ or an alkylene group, preferably of from 1 to 4 carbon atoms, more preferably of from 2

to 3 carbon atoms, but at least one of $R^7$ is $R^5$.

Preferred compounds of Formula 2 include 2,4-di-oxo-1,5,7,12-tetraazacyclotetradecane-N,N',N'',N'''-tetraacetic acid.

Compounds of Formula 2 are prepared by methods described in I. Tabushi et al. in Tetrahedron Letters, #12, pp. 1049-1052, 1977. For example, 1,3-(2'-aminoethylamino)propane can be condensed with diethyl malonate in ethanol under reflux for 3 days to give 2,4-dioxo-1,5,8,12-tetraazatetradecane which can be isolated by silica gel chromatography, eluting with 5:1 chloroform/methanol. This cyclic diamide can then be alkylated in aqueous solution at pH = 9 using bromoacetic acid at room temperature to give the tetraacetic acid derivative of the diamide [2,4-dioxo-1,5,5,12-tetraazacyclotetradecanetetraacetic acid].

Alternatively, the preparation of cyclic amide chelating agents of Formula 2 disclosed by J. Richard Morphy et al. in J. Chem. Soc., Chem. Commun. page 156-158, 1988 and T.J. Collins and E.S. Uffelman in Angew, Chem. Int. Ed. Engl. vol 28(11), pages 1509-1511, 1989, by C.F. Meares et al. in U.S. Patent 4,678,667 (July 7, 1987) and I.Tabushi et al in Tetrahedron Letters #12, p. 1049-1052, 1977 can be used to prepare such cyclic amides as 2,4-di-oxo-1,4,7,10-tetraazocyclotridecane which can then be alkylated with bromoacetic acid to make 2,4-di-oxo-1,4,7,10-tetraazqcyclotridecane- N,N',N'',N'''-tetraacetic acid.

The compounds of the invention are effective as chelants. Effectiveness as a chelant is conveniently measured by complexing the chelant with a metal such as copper such as by mixing an aqueous solution of known concentration of the chelant with an aqueous solution containing copper (II) ions of known concentration and measuring chelation capacity by titrating the chelant with copper in the presence of an indicator dye, using as an endpoint detector a photosensitive electrode.

The stability constant may be calculated by comparing potentiometric pH measurement of the chelant in the absence of and in the presence of known concentrations of metal ion as described in DETERMINATION AND USE OF STABILITY CONSTANTS by Martell and Motekaitis, VCH Publishers, 1985, pp. 14 and 21-27. Various methods may be employed to determine stability constant. Preferably, the compounds are at least as effective as NTA or EDTA.

Chelating capacity is not, however, a direct indicator of effectiveness in activities such as stabilizing bleach. For instance, hydroxyethyliminodiacetic acid (HEIDA) is effective in chelating, for example copper (358 mg copper per gram of chelant), but is relatively ineffective in stabilizing bleaches. Tests of relative effectiveness are conducted in solutions simulating cleaning formulations having bleaches and metal ions such as those tests described in the examples of the invention. In addition to chelating ability, a chelant must be resistant to degradation by or reaction with the bleaching agent to effectively stabilize a bleach or bleaching composition.

Compounds of the invention are preferably biodegradable. Biodegradability is indicated by degradation on exposure to bacteria. Standardized tests such as ASTM D-2667-82 are preferably used to determine biodegradability. In that test, a standardized sludge containing municipal waste treatment plant organisms is used to biodegrade the chelant in the presence of metal ions representative of those found in the environment including iron. Such a test simulates the environment encountered in a municipal waste treatment plant for screening the inherent biodegradability of non-volatile, water-soluble compounds.

The compounds of this invention are generally employed in the form of a water-soluble salts, notably alkali metal salts, ammonium salts, or alkyl ammonium salts. The alkali metal salts can involve one or a mixture of alkali metal salts although the potassium or sodium salts, especially the partial or complete sodium salts of the acids of Formula 1 are preferred because of their relatively low cost and enhanced effectiveness. Because the detergent formulations are generally used in alkaline aqueous systems, it is entirely feasible to use in their manufacture either the acids of Formula 1 or 2 themselves or the partially neutralized free acids. The free acid groups(s) will be converted to the appropriate salt at least as soon as the formulations are put to use in an alkaline environment.

Chelants of the invention are useful, for instance, in food products vulnerable to metal-catalyzed spoilage or discoloration; in cleaning and laundering products for removing metal ions, for example from hard water, that may reduce the effectiveness, appearance, stability, rinsibility, bleaching effectiveness, germicidal effectiveness or other property of the cleaning agents; in personal care products like creams, lotions, deodorants and ointments to avoid metal-catalyzed oxidation and rancidity, turbidity, and reduced shelf-life; in pulp and paper processing to enhance or maintain bleaching effectiveness; in pipes, vessels, heat exchangers, evaporators, filters to avoid or remove scaling, in pharmaceuticals; in metal working; in textile preparation, desizing, scouring, bleaching, and dyeing; in agriculture as in chelated micronutrients or herbicides; in polymerization or stabilization of polymers; in photography, for example in developers or bleaches; in the oil field such as for drilling, production, recovery, and hydrogen sulfide abatement.

In detergent compositions, bleach compositions, cleaning compositions and sequestrant (chelating agent) compositions, the chelants of the invention can be used to control the level of free heavy metal ions

in the compositions themselves and in liquors for example wash liquors, prepared therefrom. The amount used, if used as a chelant, is advantageously from 0.01 to 40 weight per cent, based on the total weight of the detergent constituents. The compositions generally comprise from 1 to 99.99, preferably from 5 to 30 weight percent detergent; optionally, from 5 to 40 weight percent builder; and, optionally, from 3 to 30 weight percent bleach.

Their advantageous action also includes bleaching agent stabilization, for example for sodium perborate, in detergents and in the bleaching of textiles, pulp or paper stock. Traces of heavy metals, such as iron, copper and manganese, are present in the washing powder itself, in the water and in the textile or pulp material, and they catalyze the decomposition of the sodium perborate or other bleaches. The chelants according to the invention bind these metal ions and prevent the undesirable decomposition of the bleaching system during storage and in the wash liquor. This enhances the efficiency of the bleaching system and reduces fiber damage.

In addition, enzymes, optical brighteners and scents are advantageously protected from heavy metal catalyzed oxidative decomposition.

In liquid cleaning formulations the novel chelants can be used as preservatives advantageously in an amount from 0.05 to 15 percent by weight, based on the total weight of the formulation.

In soaps the novel chelants prevent, for example, metal catalyzed oxidative decompositions.

Furthermore, they give excellent performance in detergents as builders for preventing precipitates and incrustations on the fabric.

The chelants can be used in industrial processes whenever precipitates of Ca, Mg and heavy metal salts are a nuisance and are to be prevented. They are used, for example, for preventing scale deposits and incrustations in kettles, pipelines, spray nozzles or generally on smooth surfaces.

They are suitably used for stabilizing phosphates in alkaline degreasing baths and to prevent the precipitation of lime soaps and as a result prevent the tarnishing of nonferrous surfaces and prolong the service lives of alkaline cleaning baths.

They can be used as chelants in alkaline derusting and descaling baths and also in electroplating baths and also in electroplating baths in place of cyanides as sequestrants of impurities.

The treatment of cooling water with the novel chelants prevents and redissolves scale deposits. Of advantage is the use in an alkaline medium, thereby removing corrosion problems.

In the polymerization of rubber the chelants of the invention are suitably used for preparing for example the redox catalysts used therein. They additionally prevent the precipitation of such compounds as iron hydroxide in an alkaline polymerization medium.

In the photographic industry, the novel chelants are suitably used in developer/fixing baths made up with hard water to alleviate precipitations that lead to fogging on film and photographs and alleviate deposits in the tanks. Iron(III)-complexing solutions are advantageously used in bleach fixing baths to replace less safe solutions.

In the textile industry, the chelants are suitably used for removing heavy metal traces during the manufacture and dyeing of natural and synthetic fibers, thereby preventing many problems, such as dirt spots and stripes on the textile material, loss of luster, poor wettability, unlevelness and off-shade dyeings.

In the paper industry, the chelants are suitably used for eliminating heavy metal/iron ions. Iron deposits on paper lead to hot spots where oxidative, catalytic decomposition of the cellulose starts.

Exemplary of various other uses are applications in pharmaceuticals, cosmetics and foodstuffs where metal catalyzed oxidation of olefinic double bonds and hence rancidification of goods is prevented. The chelates are also useful as catalysts for organic syntheses (for example air oxidation of paraffins, hydroformylation of olefins to alcohols).

In plant nutrition, metal deficiencies are remedied by using Cu, Fe, Mn, Zn complexes. Metal are added as chelates to prevent their precipitation in the form of biologically inactive, insoluble salts.

Further fields of application for the novel chelants are gas washing, conditioning or scrubbing (of for example flue, geothermal, sour, synthesis, process, fuel, or hydrocarbon gas) to remove at least one acidic gas, preferably the removal of NOx from flue gases, $H_2S$ oxidation and metal extraction. Polyvalent metal chelates of the invention are particularly useful in removing $H_2S$ from a fluid, particularly a gas, containing $H_2S$, by (directly or indirectly) contacting the fluid with at least one chelate of at least one, preferably one polyvalent metal in a higher valence state such that sulfur is formed along with the chelate of the metal in a lower valence state. The chelate of any oxidizing polyvalent metal capable of being reduced by reaction with $H_2S$ or hydrosulfide and/or sulfide ions and, preferably which can be regenerated by oxidation, is suitable. Preferably the chelates are water soluble. Exemplary metals include lead, mercury, nickel, chromium, cobalt, tungsten, tin, vanadium, titanium, tantalum, platinum, palladium, zirconium, molybdenum, preferably iron, copper, or manganese, most preferably iron.

Chelates of the invention are suitably used in any process of removal of $H_2S$ within the skill in the art such as those exemplified by United States Patents 4,421,733; 4,614,644; 4,629,608; 4,683,076; 4,696,802; 4,774,071; 4,816,238; and 4,830,838, which are incorporated by reference herein. The polyvalent metal chelates are readily formed in aqueous solution by reaction of an appropriate salt, oxide or hydroxide of the polyvalent metal and the chelating agent in the acid form or an alkali metal or ammonium salt thereof.

Preferably contact of $H_2S$, hydrosulfide, and/or sulfide with the chelate takes place at a pH of from 6 to 10. The more preferred range is from 6.5 to 9 and the most preferred range of pH is from 7 to 9. In general, operation at the highest portion of the range is preferred in order to operate at a high efficiency of hydrogen sulfide absorption. Since the hydrogen sulfide is an acid gas, there is a tendency for the hydrogen sulfide to lower the pH of the aqueous alkaline solution. Lower pH is preferable in the presence of carbon dioxide to reduce absorption thereof. Optimum pH also depends upon stability of a particular polyvalent metal chelate. At the pH values below 6 the efficiency of hydrogen sulfide absorption is so low so as to be generally impractical. At pH values greater than 10, for instance with iron as the polyvalent metal, the precipitation of insoluble iron hydroxide may occur resulting in decomposition of the iron chelate. Those skilled in the art can ascertain a preferred pH for each operating situation.

Buffering agents optionally useful as components of aqueous alkaline scrubbing solutions of the invention include those which are capable of maintaining the aqueous alkaline solution at a pH generally in a operating pH range of 6 to 10. The buffering agents are advantageously water soluble at the concentration in which they are effective. Examples of suitable buffering agents include the ammonium or alkali metal salts of carbonates, bicarbonates, or borates, including sodium carbonate, bicarbonate or sodium borate, particularly carbonates and bicarbonates when used in the presence of $CO_2$ (carbon dioxide).

The temperatures employed in a contacting or absorption-contact zone are not generally critical, except that the reaction is carried out below the melting point of sulfur. In many commercial applications, absorption at ambient temperatures is desired. In general, temperatures from 10°C to 80°C are suitable, and temperatures from 20°C to 45°C are preferred. Contact times will range from 1 second to 270 seconds or longer, with contact times of 2 seconds to 120 seconds being preferred.

Pressure conditions suitably vary widely, depending on the pressure of the gas to be treated. For example, pressures in a contacting zone may vary from one atmosphere (100 kPa) up to one hundred fifty (15000 kPa) or even two hundred atmospheres (20000 kPa), with from one atmosphere (100 kPa) to one hundred atmospheres (10000 kPa) preferred.

In $H_2S$ removal, preferably at least an amount of chelate in a higher valence state stoichiomitric with the $H_2S$ to be removed is used. Preferred mole ratios of chelate to $H_2S$ are from 1:1 to 15:1, more preferably from 2:1 to 5:1. When chelates in both higher and lower valence states are present, it is generally preferable to maintain a concentration of lower valence state chelate at least 5 times the concentration of that in the higher valence state. When, for instance an iron chelate is used, it is preferably present in an amount from 100 to 100,0000 ppm iron in the higher valence state most preferably from 1000 to 50,000 ppm by weight iron in the higher valence state. The circulation rate of the chelate solution depends upon the hydrogen sulfide level in the $H_2S$ containing fluid. In general, the circulation rate should be sufficient to provide from 1 to 6 moles and preferably 2-4 moles of high valence (for example ferric) chelate for every mole of $H_2S$ entering the reaction zone. The contact time of the reactants should be at least 0.05 second or more and preferably in the range from 0.02 to 1.0 seconds.

Chelates of the invention are preferably used in combination with additives such as rate enhancers (or catalysts, for example for conversion of $H_2S$ to sulfur) and/or stabilizers for the chelates. Cationic polymeric catalysts are advantageous and include polyethyleneamines, poly(2-hydroxypropyl-1-N-methylammonium chloride) and the 1,1-dimethyl analogue, poly[N-(dimethylaminomethyl)acrylamide], poly(2-vinylimidazolinum bisulfate), poly(diallyldimethyl ammonium chloride) and poly(N-dimethyl aminopropyl)-methacrylamide. These cationic polymers are well known and are commercially available under various tradenames. See, for example, Commercial Organic Flocculants by J. Vostrcil et al Noyes Data Corp. 1972 which is incorporated by reference herein. Other useful cationic catalysts are set forth in J. Macromol. Science-Chem. A4 pages 1327-1417(1970) which is also incorporated by reference herein. Preferred catalysts include polyethylene amines and poly(diallyldimethyl ammonium chloride). Preferred concentration ranges for the polymeric catalysts are from 0.75 to 5.0 weight percent, and from 1.0 to 3.0 weight percent is the most preferred range. The amount of polymeric catalyst is sufficient to provide a weight ration of iron or other polyvalent metal in the range from 0.2 to 10:1. Concentrations of from 10 to 25 ppm in solution are preferred. Stabilizing agents include, for example bisulfite ions such as sodium, potassium, lithium, ammonium bisulfite and mixtures thereof. They are used in stabilizing amounts, ie. amounts sufficient to reduce or inhibit rate of degradation of the chelate, preferably from about 0.01 to about 0.6 equivalents per liter of solution, more preferably from 0.05 to 0.3 equivalents/liter.

After the chelate of lower valence state is produced from that of higher valence state, it is preferably oxidized back to the higher valence state and recycled. Oxidization is suitably by any means within the skill in the art, for example electrochemically, but preferably by contact with an oxygen-containing gas, for example air. If $CO_2$ is absorbed, it is preferably removed before contact with the oxygen-containing gas. The oxygen (in whatever form supplied) is advantageously supplied in a stoichiometric equivalent or excess with respect to the amount of lower valence state metal ion of the chelate or chelates present in the mixture. Preferably, the oxygen is supplied in an amount from 1.2 to 3 fold excess and in a concentration of from 1 percent to 100 percent by volume, more preferably from 5 percent to 25 percent by volume. Temperatures and pressures are suitably varied widely, but generally those used in the contacting zone(s) are preferred, preferably temperatures of from 10° C to 80° C more preferable from 20° C to 45° C with pressures from 0.5 atmosphere to 3 or 4 atmospheres preferred. Mild oxidizing conditions are generally preferred to avoid degradation of chelating agent. Such conditions are within the skill in the art.

Sulfur produced by reaction of $H_2S$ with the polyvalent metal chelate is optionally solubilized, for example by oxidation. Oxidation is suitably by any means within the skill in the art. When $SO_2$ is present or easily generated by oxidation of $H_2S$ (for example using oxygen or electrochemical means)it is a preferred oxidizing agent to produce, for example thiosulfates from the sulfur. Other suitable oxidizing agents include for example alkali metal or ammonium salts of inorganic oxidizing acids such as perchloric, chloric, hypochlorous, and permanganic acids. Otherwise, the sulfur is optionally recovered by means within the skill in the art including flocculation, settling, centrifugation, filtration, and flotation.

Processes of the invention include, for instance: a process for removing at least a portion of $H_2S$ from a fluid stream containing $H_2S$ which comprises (A) contacting said fluid stream (optionally in a first reaction zone) with an aqueous solution at a pH range suitable for removing $H_2S$ wherein said solution comprises at least one higher valence polyvalent metal chelate of Formula 1 wherby said higher valence polyvalent metal chelate is reduced to a lower valence polyvalent metal chelate. Optionally the aqueous solution additionally comprises an oxidizing agent capable of oxidizing elemental sulfur to soluble sulfur compounds, and/or one or more water soluble cationic polymeric catalysts and/or a stabilizing amount of a stabilizing agent each as bisulfite ion.

The process optionally includes at least one additional step such as:

(B) contacting said solution containing the lower valence polyvalent chelate in a second reaction zone with an oxygen-containing gas stream whereby said chelate is reoxidized;

(C) recirculating said reoxidized solution back to said first reaction zone;

(D) feeding said aqueous solution from said oxidation zone to a sulfur recovery zone;

(E) removing from said aqueous solution at least a portion of said sulfur and thereafter;

(F) regenerating the aqueous admixture in a regeneration zone to produce a regenerated reactant;

(G) returning aqueous admixture containing regenerated reactant from the regeneration zone to the contacting zone;

(H) incinerating hydrogen sulfide to form sulfur dioxide;

(I) selectively absorbing said sulfur dioxide in an alkaline aqueous solution without substantial carbon dioxide absorption to form a solution of sulfites essentially free of insoluble carbonates;

(J) contacting said sulfur with said sulfites to form soluble sulfur compounds;

(K)recirculating said reoxidized polyvalent metal chelate back to said fluid stream/aqueous chelate solution contacting step; and/or

(L)condensing geothermal steam in a reaction zone, preferably in said first reaction zone, for contacting said reduced polyvalent metal chelate.

Compositions of the invention, thus, include aqueous solutions of polyvalent metal chelates of the invention (in one or more oxidation states) with at least one of: $H_2S$, sulfide or bisulfide ions, rate enhancers such as poly(dimethyldiallyl ammonium chloride) and/or polyethyleneamines, and/or stabilizers such as bisulfite ions.

Similarly, chelates of the invention are used in removal of nitrogen oxides, preferably nitric oxide (NO), from fluids containing them. For instance, nitrogen oxides ($NO_x$) and $SO_2$ can be removed from flue gas streams by absorbing the $SO_2$ using an absorbent or reactant therefor, particularly an amine based absorbent such as a nitrogen-containing heterocyclic compound preferably having at least one carbonyl group such as a piperazinone; piperidinone, piperidine, piperazine or triazine having a carbonyl group; hydantoin; cyclic urea, oxazolidone or morpholinone in conjunction with a chelate of a polyvalent metal. Representative metal ions are chromium, cobalt, copper, iron, lead, manganese, mercury, molydenum, nickel, palladium, platinum tin, titanium, tungsten, and vanadium; preferably iron, copper, and/or nickel all preferably with a valence of +2, the more preferably iron, most preferably iron in the ferrous state. Such chelates are conveniently prepared by admixing a water soluble salt of the metal, such as a sulfate, acetate,

or oxalate, with a water soluble form of the cheating agent, for example a salt, advantageously in water. The chelates are useful in any process within the skill in the art such as those disclosed in United States Patents 4,732,744 to Chang et al.; 4,612,175 to Harkness et al.; 4,708,854 to Grinstead; 4,615,780 to Walker; 4,126,529 to DeBerry; 4,820,391 to Walker; and 4,957,716 to Cichanowicz et al. When an SO2 absorbent is used, it is preferably regenerated, more preferably thermally regenerated, and preferably recycled. The concentration of NOX in the fluid (directly or indirectly) contacting the chelate is preferably from 1 ppm to 15,000 ppm by volume such as is found, for instance, in flue gases from burning for example coal.

Whether used with an absorbent for $SO_2$ or not, the metal chelate is advantageously present in the solution which contacts the $NO_x$ containing fluid at a metal ion concentration greater than 100 ppm with a chelating agent to metal ion molecular ratio of greater than or equal to one. The metal chelate is preferably present at a metal ion concentration of 1,000 to 10,000 ppm and a chelating agent to metal on molecular ratio between 1:1 and 10:1. The optimum amounts depend on the chelating agent generally with preferred ratios between 1:1 and to 5:1.

An absorber is suitably operated at a temperature of from 0° to 120°C, but is preferably operated at a temperature of from 5° to 95°C. In the process, both absorber and (optionally) a stripper are typically operated at a pressure of from atmospheric to 10 atmospheres (for example 0 to 900 kPa gauge), however, atmospheric pressure is preferred for the convenience of lower equipment and operating costs and reduced $SO_2$ absorbent losses. Higher temperatures and pressures are not deleterious so long as they are below the decomposition temperature of the chelate and absorbent, if present. The absorber is preferably maintained at a pH between 3 and 8 to retain $NO_x$ absorbence in the absorber.

Chelates absorb $NO_x$ or act as stoichiometric reactants to increase the solubility of of $NO_x$ in aqueous solution. Preferably sulfite and/or bisulfite ions collectively referred to herein as "sulfites" are also present. Such ions react with the $NO_x$-chelate complex to form iminodisulfonate salts and free the chelate for $NO_x$ absorption. Examples of suitable soluble sulfite salts include sodium, potassium, lithium, magnesium and/or ammonium sulfite and/or bisulfite. When $SO_2$ is present, $SO_2$ in aqueous solution forms sulfurous acid, and the concentration of sulfites in the absorbent is generally sufficient for iminodisulfonate formation without replenishment, but sulfites may be added, if necessary, to maintain a concentration of at least 0.05 to 1 g-moles/l absorbent, preferably at least 0.1 g-moles/l. A sulfite salt is, thus, preferably present with the chelate.

Alternatively, as described in U.S Patent 4,957,716, which is incorporated herein by reference in its entirety, the chelate promotes absorption of $NO_x$ which may be converted to such compounds as $HNO_2$ and $HNO_3$ which react with $HSO_3$, if present, to form hydroxylaminedisulfonate ($HON(SO_3H)_2$, abbreviated HADS) and related compounds, which are preferably subsequently converted to soluble ammonium and sulfate ions advantageously at a pH of 4.2 or less, preferably 4. More preferably the ammonium ions are subsequently removed, for example by absorption, and most preferably, the sulfate ions are precipitated

In removing $NO_x$ from a fluid, the polyvalent metal chelate is oxidized from a lower to a higher valence state. The lower valence metal chelate is preferably replenished, for example by replacement of the polyvalent metal ion of the chelate, but more preferably by reduction of the metal by any means within the skill in the art, such as by contact with a reducing agent, or preferably by electrochemical means (at a cathode). The chelate is, then, preferably recycled.

When electrochemical regeneration is used, the solution containing the higher valence polyvalent metal chelate (which solution is preferably first (advantageously thermally) stripped of $SO_2$) is preferably directed to a cathode compartment of an electrochemical cell comprised of an anode in an anode compartment separated, preferably by a membrane, from a cathode in a cathode compartment. An electrical potential is imposed across the anode and cathode to reduce inactive oxidized chelates to an active state. Preferably, an anionic exchange membrane is used. Heat stable amine salts may also be converted to free amine sorbent in the cathode compartment and soluble salt anions diffuse from the cathode compartment through the anion exchange membrane into the anode department. Preferably, in a further step, regenerated absorbent solution from the cathode compartment is recycled to the NOx containing fluid contacting step. The process more preferably additionally comprises a step of adjusting the pH of the regenerated recycle absorbent to from 3 to 8.

Compositions of the invention, thus, include aqueous solutions of the polyvalent metal chelates of the invention with at least one of $NO_X$, at least one (water soluble) sulfite, or at least one absorbent for $SO_2$. Mixtures of the chelates in higher and lower valence states and mixtures of the chelate with the chelate -$NO_X$ complex are also aspects of the instant invention.

Processes of the invention, thus, include a process for removing at least a portion of $NO_X$, preferably NO, from a fluid containing $NO_X$, said fluid preferably also containing $SO_2$ and said fluid preferably being a gas, but suitably being a liquid, suspension, and condensate comprising the step of

(A) (directly or indirectly) contacting the fluid with an aqueous solution comprising at feast one lower valence state polyvalent metal chelate of the invention and optionally additionally containing an absorbent for $SO_2$ and/or a sulfite.

The process optionally additionally comprises at least one of the following steps:

(B)thermally stripping sulfur dioxide from an $SO_2$-rich absorbent solution to obtain an $SO_2$-lean absorbent solution;

(C) directing the absorbent solution to a cathode compartment in an electrochemical cell, said cell having an anode in an anode compartment separated (preferably by a membrane) from a cathode in said cathode compartment, and imposing an electrical potential across said anode and said cathode to reduce oxidized chelates in said cathode compartment to obtain a regenerated absorbent solution;

(D) recycling said regenerated absorbent solution to contacting step (A);

(E) converting heat stable amine salts into free amine absorbent in said cathode compartment;

(F) separating salt anions from said cathode compartment through said anionic exchange membrane into said anode compartment;

(G) circulating an aqueous electrolyte solution through said anode compartment;

(H) periodically refreshing said electrolyte to eliminate byproduct salts in said anode compartment;

(I) adjusting said regenerated absorbent solution to a pH of from 3 to 8 for a recycling step;

(J)(when HADS is formed) mixing at least a portion of hydroxylaminedisulfonate in a reaction zone in an aqueous environment of pH of 4.2 or less, thereby converting said hydroxylaminedisulfonate to ammonium ions and sulfate ions in a second aqueous solution;

(K) contacting said second aqueous solution with a second ammonium ion-absorbing sorbent suitable for removing ammonium ions from said second aqueous solution and separating said second sorbent from said second aqueous solution;

(L) eluting said second sorbent and exposing the eluted ammonium ions or ammonia to nitrogen oxides at a temperature sufficient to form nitrogen and water therefrom; and/or

(M) removing said sulfate ions from said second aqueous solution by forming a sulfate salt precipitate.

The chelants for alkaline earth metal and heavy metal ions according to the invention are used as complexing agents in general and specifically in detergents and also rinse and wash assistants, in particular as complexing agents for heavy metal and/or alkaline earth metal ions, as bleaching agent stabilizers and as builders.

The present invention accordingly provides the corresponding uses and detergents which contain these compounds as well as the constituents known to those skilled in the art.

The compounds to be used according to the invention are used in cleaning formulations, particularly detergent, in general in an amount from 0.01 to 40 weight percent, preferably from 0.05 to 20 weight percent, more preferably from 0.1 to 10 weight percent based on the total weight of the detergent formulation.

If specifically used as a builder, amounts from 1 to 40 percent by weight are particularly preferred, while if specifically used as a bleaching agent stabilizer for perborates, or other bleaches such as sources of hydrogen peroxide or oxygen including percarbonates, peroxides (for example hydrogen peroxide or sodium peroxide), persulfate, perthalates, and per acid precursors (for example tetraacetyl-ethylene diamine), amounts from 0.05 to 1 percent by weight are preferred. If used specifically as a chelant in detergents, amounts from 0.01 to 2 percent by weight are preferred. Chelants of the invention are particularly useful in stabilizing bleach, particularly peroxide beaches, more particularly perborates.

As builders, the chelants of this invention can be advantageously used with a wide variety of detergent actives or surfactants, including those known in the art as anionic, cationic, nonionic, ampholytic, and zwitterionic detergents as well as any suitable mixture of such detergents. When the resultant washing compositions are used in aqueous washing systems, the cleaning power of the formulation is enhanced in much the same way as when the commonly used polyphosphate builders are employed. Yet the present builder systems are more favorably degraded than the polyphosphates and do not contribute to the eutrophication problems characteristic of phosphorus-containing builders.

Accordingly, this invention provides, inter alia, a washing composition composed of an organic detergent surfactant suitable for use in water and, as a builder, a water-soluble salt of at least one acid of Formula 1 or 2. Although the proportions maybe varied to suit the needs of the occasion, the weight ratio of the detergent surfactant to the builder of this invention will normally fall within the range of 100:1 to 1:10.

As builders the compounds of this invention are generally employed in the form of a water-soluble salts, notably alkali metal salts, ammonium salts, or alkyl ammonium salts. The alkali metal salts can involve one or a mixture of alkali metal salts although the potassium or sodium salts, especially the tetrasodium salt of the acids of Formula 1 or 2 are preferred because of their relatively low cost and enhanced effectiveness.

Because the detergent formulations are generally used in alkaline aqueous systems, it is entirely feasible to use in their manufacture either the acids of Formula 1 or 2 themselves or the partially neutralized free acids. The free acid group(s) will be converted to the appropriate salt at least as soon as the formulations are put to use in an alkaline environment.

For best results, the formulations of this invention wherein compounds of the invention are used as builders will provide in aqueous solution a pH between 8 and 12.

Detergent formulations which, based on the total weight, contain from 0.01 to 40, preferably from 0.05 to 20 percent by weight of compound to be used according to the invention generally contain as additional constituents, based on the total weight, from 6 to 25 percent by weight of surfactants, from 15 to 50 percent by weight of builders with or without cobuilders, from 0 to 35 percent by weight of bleaching agents with or without bleaching agent activators, and from 3 to 30 percent by weight of assistants, such as enzymes, foam regulants, corrosion inhibitors, optical brighteners, scents, dyes or formulation aids, eg. sodium sulfate.

The compounds of this invention can be used with a wide variety of detergents including those classed in the art as anionic detergents, cationic detergents, nonionic detergents, ampholytic (i.e., amphoteric) detergents, and zwitterionic detergents, and any suitable mixture of two or more of these (whether from the same class or from different classes). The chelants of this invention perform particularly well with anionic or nonionic surface-active compounds and therefore this constitutes a preferred embodiment of the invention.

Another preferred embodiment of this invention is a washing composition comprising an organic detergent surfactant, at least one water-soluble salt of an acid of Formula 1 or 2 and to 10 percent by weight based on the total weight of the composition of a water-soluble alkali metal silicate. The cleaning efficiency of these preferred compositions is at least comparable to commercially available household and laundry formulations. Moreover, the soluble silicates of such alkali metals as sodium and potassium serve as effective corrosion inhibitors. In accordance with this preferred embodiment it is desirable to employ one or more silicates of sodium or potassium, or both, wherein the weight ratio of $SiO_2:M_2O$(M = Na or K) is in the range of from 1:1 to 2.8:1. Sodium silicates wherein this ratio is in the range of from 1.6:1 to 2.5:1 are especially useful because of their low cost and effectiveness.

Another preferred embodiment of this invention involves including with the mixture of the organic detergent surfactant and the compounds of the invention (e.g., the tetrasodium salt, the tetrapotassium salt, or the mixed sodium-potassium salts-including the partial and complete salts thereof), an alkali metal sulfate, preferably sodium sulfate, or an alkali metal carbonate, preferably sodium carbonate, or both Amounts of the compounds of the invention up to 60 percent by weight of the total formulation are suitable. These formulations are effective detergent formulations for laundry, household and/or industrial use. In the preferred compositions the amount of alkali metal sulfate and/or alkali metal carbonate is generally from 10 to 50 percent by weight based on the total weight of the formulation.

In a particularly preferred embodiment, compounds of Formula 1 or 2 (chelants) are used with surfactants in compositions additionally containing a bleaching agent, preferably a bleaching agent which supplies a peroxide ion in water such as hydrogen peroxide, a perborate salt, percarbonate salt or shelf-stable form of peroxide, more preferably the bleaching agent is a perborate salt or hydrogen peroxide, most preferably the bleaching agent is sodium perborate or hydrogen peroxide (in aqueous solution). While the chelants are present in a weight ratio of from 1:40 to 10:1, preferably from 1:5 to 3:2 relative to the surfactant; the bleaching agent is present in a ratio of from 150:1 to 2:1, preferably from 60:1 to 20:1 relative to the bleach stabilizer; in a solution containing bleaching agent, the bleaching agent is advantageously present in an amount of from 2 to 50, preferably from 10 to 30 weight percent based on dry detergent formulation. In bleaching compositions having little or no surfactant, such as those for all fabric bleaches or peroxy bleach additives, the chelants are preferably present in amount of from 0.05 to 5 percent by weight relative to the bleaching agent present.

Compounds of the invention are also advantageously used in cleaning compositions, particularly laundry compositions, having low phosphate. For instance, a typical dry phosphate detergent composition would have from 5 to 50 percent phosphate and from 0 to 10 by weight chelant. Chelants of the invention are preferably used in compositions having less than 40 weight percent phosphate, more preferably less than 30, most preferably less than 20, even more preferably less than 10 weight percent phosphate. Such compositions preferably contain from 0.2 to 20 weight percent chelant, more preferably from 0.5 to 5 by weight chelant.

Chelants of the invention are also useful in surfactant-free cleaning compositions including built cleaning compositions suitable for hard-surface cleaning, such as certain automatic diswhashing agents and kitchen or bathroom cleaners. Such cleaning compositions generally comprise from 1 percent to 99.95 percent, preferably 90 percent to 99 weight percent, of a conventional builder and at least 0.5 weight percent, typically 0.1 to 5 weight percent chelant.

The compounds according to the invention can also be used as complexing agents, builders and bleaching agent stabilizers in detergent formulations together with other, prior art agents, in which case the general properties can be substantially improved in respect of sequestration, incrustation inhibition, grayness inhibition, primary washing action and bleaching action.

Suitable surfactants for use in the cleaning compositions of the invention are those which contain in the molecule one or more hydrophobic organic radicals and one or more water-solubilizing anionic, zwitterionic or nonionic groups. The hydrophobic radicals usually are aliphatic hydrocarbyl of 8 to 26, preferably 10 to 22, in particular 12 to 18, carbon atomsor aromatic alkyl having 6 to 18, preferably 8 to 16, aliphatic carbon atoms.

Suitable synthetic anionic surfactants are in particular those of the sulfonate, sulfate or synthetic carboxylate type.

Suitable builder substances are for example: wash alkalis, such as sodium carbonate and sodium silicate, or complexing agents, such as phosphates, or ion exchangers, such as zeolites, and mixtures thereof. These builder substances have as their function to eliminate the hardness ions, which come partialy from the water, partialy from dirt or textile material, and to support the surfactant action. In addition to the above mentioned builder substances, the builder component may further contain cobuilders. In modern detergents, it is the function of cobuilders to undertake some of the functions of phosphates, eg. sequestration, soil antiredepositon and primary and secondary washing action.

The builder components may contain for example water-insoluble silicates as described for example in German Laid-Open Application DE-OS No. 2,412,837 and/or phosphates. As phosphate it is possible to use pyrophosphates, triphosphates, higher polyphosphates and metaphophates. Similarly, phosphorus-containing organic complexing agents such as alkanepolyphosphonic acids, amino- and hydroxy-alkanepolyphosphonic acids and phosphonocarboxylic acids, are suitable for use as further detergent ingredients generally referred to as stabilizers or phosphonates. Examples of such detergent additives are the following compounds: methanediphophonic acid, propane-1,2,3-triphosphonic acid, butane-1,2,3,4-tetraphosphonic acid, polyvinylphosphonic acid, 1-aminoethane,-1,1-diphosphonic acid, aminotris-methylenetriphosphonic acid, methylamino- or ethylaminobismethylenediphosphonic acid, ethylenediaminetetramethylenephosphonic acid, diethylenetriaminopentamethylenephosphonic acid, 1-hydroxyethane-1,1-diphosphonic acid, phosphonoacetic and phosphonopropionic acid, copolymers of vinylphosphonic acid and acrylic and/or maleic acid and also partially or completely neutralized salts thereof.

Further organic compounds which act as chelants for calcium and may be present in detergent formulations are polycarboxylic acids, hydroxcarboxylic acids and aminocarboxylic acids which are usually used in the form of their water-soluble salts.

Examples of polycarboxylic acids are dicarboxylic acids of the general formula HOOC-$(CH_2)$m-COOH where m is 0-8, and also maleic acid, methylenemalonic acid, citraconic acid, mesaconic acid, itaconic acid, noncyclic polycarboxylic acids having 3 or more carboxyl groups in the molecule, for example tricarballylic acid, aconitic acid, ethylenetetracarboxylic acid, 1,1,3-propanetetracarboxylic acid, 1,1,3,3,5,5-pentanehexacarboxylic acid, hexanehexacarboxylic acid, cyclic di- or poly-carboxylic acids, for example cyclopentanetetracarboxylic acid, cyclohexanehexacarboxylic acid, tetrahydrofurantetracarboxylic acid, phthalic acid, terephthalic acid, benzene-tricarboxylic, -tetra-carboxylic or -pentacarboxylic acid and mellitic acid.

Examples of hydroxymonocarboxylic and hydroxypolycarboxylic acids are glycollic acid, lactic acid, malic acid, tartronic acid, methyltartronic acid, gluconic acid, glyceric acid, citric acid, tartaric acid and salicylic acid.

Examples of aminocarboxylic acids are glycine, glycylglycine, alanine, asparagine, glutamic acid, aminobenzoic acid, iminodiacetic acid, iminotriacetic acid, hydroxyethyliminodiacetic acid, ethylenediaminetetraacetic acid, hydroxyethylethylenediaminetriacetic acid, diethylenetriaminepentaacetic acid and higher homologues which are preparable by polymerization of an N-aziridylcarboxylic acid derivative, for example of acetic acid, succinic acid or tricarballylic acid, and subsequent hydrolysis, or by condensation of polyamines having a molecular weight of from 500 to 10,000 with salts of chloroacetic or bromoacetic acid.

Preferred cobuilder substances are polymeric carboxylates. These polymeric carboxylic acids shall include the carboxymethyl ethers of sugars, of starch and of cellulose. Zeolites and phosphates are also useful.

Particularly important polymeric carboxylic acids are for example the polymers of acrylic acid, maleic acid, itaconic acid, mesaconic acid, aconitic acid, methylenemalonic acid, and citraconic acid, the copolymers between the aforementioned carboxylic acids, for example a copolymer of acrylic acid and maleic acid in a ration of 70:30 and having a molecular weight of 70,000, or copolymers thereof with ethylenically unsaturated compounds, such as ethylene, propylene, isobutylene, vinyl methyl ether, furan,

acrolein, vinyl acetate, acrylamide, acrylonitrile methacrylic acid, and crotonic acid, for example the 1:1 copolymers of maleic anhydride and methyl vinyl ether having a molecular weight of 70,000 or the copolymers of maleic anhydride and ethylene and/or or propylene and/or furan.

The cobuilders may further contain soil antiredeposition agents which keep the dirt detached from the fiber in suspension in the liquid and thus inhibit graying. Suitable for this purpose are water-soluble colloids usually of an organic nature for example the water-soluble salts of polymeric carboxylic acids, glue, gelatin, salts of ethercarboxylic acids or ethersulfonic acids of starch and of cellulose or salts of acid sulfates of cellulose and of starch. Even water-soluble polyamides containing acid groups are suitable for this purpose. It is also possible to use soluble starch products and starch products other than those mentioned above, for example degraded starch, and aldehyde starches. Polyvinylpyrrolidone is also usable.

Bleaching agents are in particular hydrogen peroxide and derivatives thereof or available chlorine compounds. Of the bleaching agent compounds which provide $H_2O_2$ in water, sodium perborate hydrates, such as $NaBO_2.H_2O_2.3H_2O$ and $NaBO_2.H_2O_2$, are particular importance. However, it is also possible to use other $H_2O_2$-providing borates. These compounds can be replaced in part or in full by other sources of active oxygen, in particular by peroxyhydrates, such as peroxycarbonates, peroxyphosphonates, citrate perhydrates, urea,-$H_2O_2$-providing peracid salts, for example caroates, perbenzoates or peroxyphthalates or other peroxy compounds.

Aside from those according to the invention, customary water-soluble and/or water-insoluble stabilizers for peroxy compounds can be incorporated together with the former in amounts from 0.25 to 10 percent by weight, based on the peroxy compound. Suitable water-insoluble stabilizers are the magnesium silicates $MgO:SiO_2$ from 4:1 to 1:4, preferably from 2:1 to 1:2, in particular 1:1, in composition, usually obtained by precipitation from aqueous solutions. Other alkaline earth metals of corresponding composition are also suitably used.

To obtain a satisfactory bleaching action even in washing at below 80 ° C, in particular in the range from 60 ° C to 40 ° C, it is advantageous to incorporate bleach activators in the detergent, advantageously in an amount from 5 to 30 percent by weight, based on the $H_2O_2$-providing compound.

Activators for per-compounds which provide $H_2O_2$ in water are certain N-acyl and O-acyl compounds, in particular acetyl, propionyl or benzyl compounds, which form organic peracids with $H_2O_2$ and also carbonic and pyrocarbonic esters. Useful compounds are inter alia:

N-diacylated and N,N'-tetraacylated amines, for example N,N,N',N'-tetraacetylmethylenediamine or-ethylenediamine, N,N-diacetylaniline and N,N-diacetyl-p-toluidine, and 1,3-diacylated hydantoins, alkyl-N-sulfonyl-carboxamides, N-acylated hydrazides, acylated triazoles or urazoles, for example monoacetyl-maleohydrazide, O,N,N-trisubstituted hydroxylamines, for example O-benzoyl-N,N-succinylhydroxylamine, O-acetyl-N,N-succinylhydroxylamine, O-p-methoxybenzoyl-N,N-succinyl-hydroxylamine, O-p-nitrobenzoyl-N,N-succinylhydroxylamine and O,N,N-triacetylhydroxylamine, carboxylic anhydrides, for example benzoic anhydride, m-chlorobenzoic anhydride, phthalic anhydride and 4-chlorophthalic anhydride, sugar esters, for example glucose pentaacetate, imidazolidine derivatives, such as 1,3-diformyl-4,5-diacetoxyimidazolidine, 1,3-diacetyl-4,5-diacetoxyimidazoline and 1,3-diacetyl-4,5-dipropionyloxyimidazolidine, acylated glycolurils, for example tetrapropionylglycoluril or diacetyldibenzoylglycoluril, dialkylated 2,5-diketopiperazines, for example 1,4-dipropionyl-2,5-diketopiperazine and 1,4-dipropionyl-3,6-dimethyl-2,5-diketopiperazine and 1,4-dipropionyl-3,6-2,5-diketopiperazine, acetylation and benzoylation products of propylenediurea or 2,2-dimethylpropylenediurea.

The sodium salt of p-(ethoxycarbonyloxy)benzoic acid and of p-(propoxycarbonyloxy) benzenesulfonic acid and also the sodium salts of alkylated or acylated phenolsulfonic esters, such as p-acetoxybenzenesulfonic acid, 2-acetoxy-5-nonylbenzenesulfonic acid, 2-acetoxy-5-propylbenzenesulfonic acid or of isononanoyloxyphenylsulfonic acid.

The bleaching agents used can also be active chlorine compounds of the inorganic or organic type. Inorganic active chlorine compounds include alkali metal hypochlorites which can be used in particular in the form of their mixed salts and adducts on orthophosphates or condensed phosphates, for example on pyrophosphates and polyphosphates or on alkali metal silicates. If the detergent contains monopersulfates and chlorides, active chlorine will form in aqueous solution.

Organic active chlorine compounds are in particular the N-chlorine compounds where one or two chlorine atoms are bonded to a nitrogen atom and where preferably the third valence of the nitrogen atom leads to a negative group, in particular to a CO or $SO_2$ group. These compounds include dichlorocyanuric and trichlorocyanuric acid and their salts, chlorinated alkylguanides or alkylbiguanides, chlorinated hydantoins and chlorinated melamines.

Examples of additional assistants are: suitable foam regulants, in particular if surfactants of the sulfonate or sulfate type are used, are surface-active carboxybetaines or sulfobetaines and also the abovementioned

nonionics of the alkylolamide type. Also suitable for this purpose are fatty alcohols or higher terminal diols.

Reduced foaming, which is desirable in particular for machine washing, is frequently obtained by combining various types of surfactants, for example sulfates and/or sulfonates, with nonionics and/or with soaps. In the case of soaps, the foam inhibition increases with the degree of saturation and the number of carbon atoms of the fatty acid ester; soaps of saturated $C_{20}$-$C_{24}$-fatty acids, therefore, are particularly suitable for use as foam inhibitors.

The nonsurfactant-like foam inhibitors include optionally chlorine-containing N-alkylated aminotriazines which are obtained by reacting 1 mole of cyanuric chloride with from 2 to 3 moles of a mono- and/or dialkylamine having 6 to 20, preferably 8 to 18, carbon atoms in the alkyl. A similar effect is possessed by propoxylated and/or butoxylated aminotriazines, for example, products obtained by addition of from 5 to 10 moles of propylene oxide onto 1 mole of melamine and further addition of from 10 to 50 moles of butylene oxide onto this propylene oxide derivative.

Other suitable nonsurfactant-like foam inhibitors are water-soluble organic compounds, such as paraffins or haloparaffins having melting points below 100 °C, aliphatic $C_{18}$- to $C_{40}$-ketones and also aliphatic carboxylic esters which, in the acid or in the alcohol moiety, possibly even both these moieties, contain not less than 18 carbon atoms (for example triglycerides or fatty acid fatty alcohol esters); they can be used in particular in combinations of surfactants of the sulfate and/or sulfonate type with soaps for foam inhibition.

The detergents may contain optical brighteners for cotton, for polyamide, for polyacrylonitrile or for polyester fabrics. Examples of suitable optical brighteners are derivatives of diaminostilbenedisulfonic acid for cotton, derivatives of 1,3-diarylpyrazolines for polyamide, quaternary salts of 7-methoxy-2-benzimidazol-2'-ylbenzofuran or of derivatives form the class of the 7-[1',2',5'-triazol-1'-yl]-3-[1'',2'',4''-triazol-1''-y]-coumarins for polyacrylonitrile. Examples of brighteners suitable for polyester are products of the class of the substituted styryls, ethylenes, thiophenes, naphthalenedicarboxylic acids or derivatives thereof, stilbenes, coumarins and naphthalimides.

It is preferred that laundry compositions herein also contain enzymes to enhance their through-the-wash cleaning performance on a variety of soils and stains. Amylase and protease enzymes suitable for use in detergents are well known in the art and in commercially available liquid and granular detergents. Commercial detersive enzymes (preferably a mixture of amylase and protease) are typically used at levels of from 0.001 to 2 weight percent, and higher, in the present cleaning compositions.

Detergent formulations of this invention may contain minor amounts of other commonly used materials in order to enhance the effectiveness or attractiveness of the product. Exemplary of such materials are soluble sodium carboxymethyl cellulose or other soil redeposition inhibitors; benzotriazole, ethylene thiourea, or other tarnish inhibitors; perfume; fluorescers; dyes or pigments; brightening agents; enzymes; water; alcohols; other builder additives, such as the water soluble salts of ethlendiaminetetraacetic acid, N-(2-hydroxyethyl)-ethylenediaminetriacetic acid; and pH adjusters, such as sodium hydroxide and potassium hydroxide. Other optional ingredients include pH regulants, polyester soil release agents, hydrotropes and gel-control agents, freeze-thaw stabilizers, bactericides, preservatives, suds control agents, fabric softeners especially clays and mixtures of clays with various amines and quaternary ammonium compounds. In the built liquid detergent formulations of this invention, the use of hydrotropic agents may be found efficacious. Suitable hydrotropes include the water-soluble alkali metal salts of toluene sulfonic acid, benzene sulfonic acid, and xylene sulfonic acid. Potassium toluene sulfonate and sodium toluene sulfonate are preferred for this use and will normally be employed in concentrates ranging up to 10 or 12 percent by weight based on the total composition.

It will be apparent from the foregoing that the compositions of this invention may be formulated according to any of the various commercially desirable forms. For example, the formulations of this invention may be provided in granular form, in liquid form, in tablet form of flakes or powders.

Use of these ingredients is within the skill in the art. Compositions are prepared using techniques with in the skill in the art.

The invention is not to be limited to any particular method of mixing the chelant and the other ingredients. The chelant may be for example mechanically mixed in the detergent in the form of a solid or slurry, or dissolved in a solution of the other ingredients. In addition, the chelant may be admixed with the other ingredient as manufactured, as well as being added simultaneously or separately to an aqueous solution. In any event, the chelant is intended to be used with the other ingredient at the time of application as a cleansing agent.

The following examples are offered to illustrate but not limit the invention. Percentages, ratios and parts are by weight unless stated otherwise. Examples of the invention (Ex.) are designated numerically, while comparative samples (C.S.), which are not examples of the invention, are designated alphabetically.

EXAMPLE 1: PREPARATION OF 1-OXO-ETHYLENEDIAMINE TETRAACETIC ACID (OXOEDTA) FROM IMIDODIACETIC ACID DISODIUM (NTA) AND NITRILOTRIACETIC ACID (IDA):

An intimate mixture of imidodiacetic acid disodium salt hydrate (IDA 5.0g, 28.2 mMole)and nitrilotriacetic acid (NTA 5.40g, 28.2 mMole) was prepared by admixing the two compounds with 150 mL of water, heating the resulting admixture to 70°C to effect solubilization, and then freeze drying the aqueous solution. The intimate mixture was isolated as 10.0230 g of a glassy solid. A2.4832 g sample of this solid was placed in a 100 mL flask under vacuum. The flask and its contents were heated to 190°C for 15 minutes during which the glassy solid becomes puffed up. The puffed up solid was cooled to room temperature and dissolved in 10 mL of water by the addition of 8 mL of 1N NaOH (sodium hydroxide). The final pH of the solution was 3.71. High Pressure Liquid Chromatography (HPLC) analysis of this solution showed the presence of OXOEDTA in 8 percent yield along with unreacted IDA and NTA as determined by peak areas of the chromatogram produced by HPLC.

The solution as then acidified with 3.86mL of 3N HCl (hydrochloric acid) and frozen. After 24 hours the solution was thawed, and the solid precipitate was filtered and dried to give 0.648 g of white solid. Analysis of the area percent of the peaks in the HPLC chromatograms of the solution before precipitation, the supernatent, and the isolated solid are given in Table 1:

Table 1

| ANALYSIS OF LOW PH SYSTEM (ultraviolet (UV)area percents of components) | | | |
|---|---|---|---|
| Component analyzed | % IDA | % NTA | % OXOEDTA |
| Whole solution | 10 | 27 | 63 |
| Supernatent(filtrate) | 15 | 22 | 64 |
| Isolated Solid | 2 | 41 | 57 |

These results indicate that the precipitate at low pH was composed of both NTA and OXOEDTA with only a trace of IDA. However, not all of the NTA and OXOEDTA were precipitated from solution.

EXAMPLES 2-3: PREPARATION OF OXOEDTA FROM THE REACTION OF IDA WITH NTA USING MICROWAVE RADIATION:

A 38 mg sample of the NTA/IDA intimate mixture (prepared as in Example 1) was placed in a tetrafluoroethylene (commercially available from DuPont de Nemours under the trade designation Teflon™) container and sealed. The sealed container was placed in a Research Microwave (commercially available from CEM Corp. under the trade designation MDS-81D) on a 6 rpm turntable for 5 minutes at 60 percent of maximum power. At the end of this time, the container was opened and the solid was dissolved in 4 mL of water containing 100 uL of 1N NaOH. HPLC analysis of this solution (pH = 3.69) indicated OXOEDTA was present.

For Comparative Sample A, a similar run was made except that the microwave oven was used at 30 percent of maximum power for 10 minutes and no OXOEDTA was obtained.

For Example 3, another run was made using the microwave oven at 60 percent of maximum power for 10 minutes; some OXOEDTA was produced, but other products began to appear. These results were summarized in Table 2 as percent of peak areas in the HPLC chromatograms of the irradiated samples:

Table 2

| HPLC ANALYSIS (AREA %) OF MICROWAVE PREPARATION OF OXOEDTA | | | | |
|---|---|---|---|---|
| Microwave Irradiation Scheme | % IDA | % NTA | % OXOEDTA | % OTHER |
| Comparative Sample A 30% Power, 5 minutes | 18 | 38 | 0 | 43 |
| Example 1 60% Power, 5 minutes | 10 | 26 | 48 | 17 |
| Example 2 60% Power, 10 minutes | 9 | 28 | 21 | 42 |

These results show that OXOEDTA can be produced by the exposure of an IDA/NTA mixture to microwave radiation.

EXAMPLE 4: HIGH pH STABILITY TEST ON OXOEDTA:

A 25.4 mg sample of OXOEDTA was prepared by the procedure of Example 16 dissolved in 5.080 mL of 0.01N NaOH and 40 uL of 25 weight percent NaOH to give a final concentration of OXOEDTA of 0.017 M at a pH of 11.85. At various time intervals, aliquots of the solution were analyzed by HPLC and the relative area percent of OXOEDTA was determined. The results are shown in Table 3:

Table 3

| HIGH pH STABILITY OF OXOEDTA | | |
|---|---|---|
| Time of high pH exposure | Area % of OXOEDTA | Area % of Other Peak(s) |
| 0 | 95 | 5 |
| 1 hour | 96 | 4 |
| 91 hours | 98 | 2 |
| 285 hours (12 days) | 98 | 2 |
| 34 days | 99 | 1 |

The results indicate that within experimental error, no significant degradation of OXOEDTA occured at a pH of 12 at room temperature within a 34 day time period.

EXAMPLES: LOW pH STABILITY TEST ON OXOEDTA

A 21.9 mg sample of OXOEDTA prepared by the procedure of Example 16 was dissolved in 3.380 mL of 0.01N HCl and 1mL water The final pH of the acidic solution of OXOEDTA was 1.71. At various time intervals, aliquots of the solution were analyzed by HPLC, and the relative area percent of OXOEDTA was determined. The results are shown in Table 5:

Table 5

| LOW pH STABILITY OF OXOEDTA | | |
|---|---|---|
| Time of Low pH exposure | Area % of OXOEDTA | Area % of Other Peak(s) |
| 0 | 95 | 5 |
| 1 hour | 95 | 5 |
| 91 hours | 96 | 4 |
| 285 hours (12days) | 93 | 7 |

The results above indicate that OXOEDTA was relatively stable in acid with only a small amount of change, believed to indicate slight hydrolysis, occurring at pH = 2 at room temperature over a 12 day period.

EXAMPLE 6: DISSOLUTION OF CALCIUM OXALATE BY OXOEDTA

A suspension was prepared by weighing out 26.28 g of calcium oxalate monohydrate and suspending it (by vigorous stirring) in 200 mL of water. Each 2 mL of this suspension contained 262.8 mg (1.8 mMole of calcium) of calcium oxalate.

A 0.09M solution of OXOEDTA was prepared by dissolving 443 mg of NaOXOEDTA prepared by the procedure of Example 20 in 10 mL of water and adding 25 mL of 3N HCl to adjust the final pH to 7.91.

Into a polypropylene tube, was placed 2 mL of the 0.09M OXOEDTA solution, 2 mL of water, and 2 mL of calcium oxalate suspension. Likewise, into a second polypropylene tube was placed 4 mL of water and 2 mL of calcium oxalate suspension. Also, into a third polypropylene tube was placed 2 mL of 3N HCl, 2 mL of water, and 2 mL of calcium oxalate suspension. All 3 tubes were capped and placed on a commercially available orbital shaker at 150 circulations per minute. After 15 minutes, all three tubes were centrifuged for 10 minutes to collect the undissolved calcium oxalate in the bottom of the tubes. A 0.1 mL aliquot of the supernatent from each tube was diluted with 9.9 mL of water and analyzed by Inductively Coupled Plasma (ICP) spectroscopy for calcium concentration. The ICP results and pH of the solutions are shown in Table 6:

Table 6

| DISSOLUTION OF CALCIUM OXALATE BY OXOEDTA | | | |
|---|---|---|---|
| Test Solution | Initial pH | Final pH | ppm Ca by ICP |
| OXOEDTA | 7.88 | 7.77 | 4.2 |
| Water | 8 | 8.15 | 0.3 |
| 3N HCl | 0.46 | 0.36 | 20 |

These results show that OXOEDTA was capable of dissolving calcium oxalate at neutral pH in a short time period relative to a water control. The amount of calcium dissolved at neutral pH was 20 percent of the calcium level produced by strong mineral acid calcium oxalate dissolution.

EXAMPLE 7: TITRATION OF OXOEDTA WITH CaCl2

A 0.01M calcium chloride solution was prepared by weighing out 292.04 mg of $CaCl_2$-$2H_2O$ (calcium chloride hydrate) and dissolving it in water in a 200 mL volumetric flask.

Into a beaker was placed a stir bar and few milligrams of Calmagite (commercially available from Aldrich Chemical Co. Inc.) indicator. To the solid was added 500 $\mu$L of 0.09 M sodium EDTA solution. A 10 mL portion of ammonium buffer was added, and the stirred solution was titrated with the 0.01N calcium chloride solution. The endpoint, as it turns from blue to purple was determined to require 4.575 mL (plus or minus 0,165 mL, in an average of 4 runs) of titrant.

Into a beaker was placed 10.4mg (30.4 $\mu$Mole) of OXOEDTA as prepared by the procedure of Example 16 which was then dissolved in 10 mL of ammonium buffer. To this solution was added a few milligrams of

Calmagite indicator. Then the resulting solution was titrated with 0.01 N calcium chloride with stirring as described above. The endpoint was determined at 2.69 mL of added titrant (average of two runs) which corresponds to 26.9 μMole of calcium per 30.4 mMole of OXOEDTA. This 0.88 calcium: OXOEDTA ratio was indicative, within experimental error, of calcium forming a 1:1 ligand to metal ratio with OXOEDTA just as calcium forms a 1:1 ligand to metal ratio with EDTA.

EXAMPLE 8: PREPARATION OF OXOEDTA FROM IDA AND GLYOXAL

A 653 mg (3.69 m Mole) portion of IDA was dissolved in 2 mL of water and treated with 52.6 uL of 40 weight per cent glyoxal in water (0.461 mMole). The 8:1 molar ratio solution of IDA:glyoxal (pH = 10.53) was then frozen and freeze-dried to give 505.3 mg of a light yellow solid.

8A. A 100 mg portion of light yellow solid was dissolved in 5 mL of water to give an aqueous solution at a pH of 10.53.

8B. A 2 mL portion of "8A" was placed in a vial in an oil bath at 90°C for 45 minutes of heat treatment. The pH after this treatment was 10.06, and the solution was a faint yellow.

8C. A 60 mg portion of the light yellow solid was placed in a glass vial and heated at 90°C for 45 minutes. The solid turned more yellow than when the heating started but was still a free flowing powder. All of the solid was dissolved in 3 mL of water to give a solution with a pH of 10.44.

8D. A 70 mg portion of the light yellow solid was placed in a glass vial and heated at 90°C for 15 hours after which it still remained as a free flowing yellow solid. All of the solid was dissolved in 3 mL of water to give a solution with a pH of 10.37.

8E. A melting point (decomposition) of 130°C was determined for the light yellow solid. A 24mg portion of the light yellow solid was put in a vial and heated at 135°C for 20 minutes. The solid turned brown and was no longer free flowing. All the solid was dissolved in 1 mL of water to give a solution with a pH of 10.43.

8F. A 26 mg portion of the light yellow solid was placed in a vial and heated at 135°C for 75 minutes. The solid turned brown and was no longer free flowing. All the solid was dissolved in 1 mL of water to give a solution with a pH of 10.40.

8G. A 31.3 mg portion of the light yellow solid was placed in a vial and heated at 135°C for 15 hours. The solid turned brown and was no longer free flowing. All the solid was dissolved in 1 mL of water to give a solution with a pH of 10.31.

All of the above solutions (8A-8G) were analyzed by HPLC for the presence of OXOEDTA. In addition, a peak with a retention time intermediate between OXOEDTA and IDA was noted in all the chromatograms and may have represented an intermediate or byproduct. This unidentified peak was termed "other". The results from the analysis of the solutions by HPLC are listed in Table 7:

Table 7

| HPLC ANALYSIS (UV AREA %) OF IDA + GLYOXAL REACTIONS | | | |
|---|---|---|---|
| Solution Analyzed | Area % of IDA | Area % of other | Area % of OXOEDTA |
| Solution from 8A | 75 | 20 | 4 |
| Solution from 8B | 67 | 27 | 6 |
| Solution from 8C | 30 | 48 | 22 |
| Solution from 8D | 31 | 50 | 19 |
| Solution from 8E | 24 | 41 | 35 |
| Solution from 8F | 23 | 42 | 35 |
| Solution from 8G | 22 | 44 | 34 |

These results indicate that mixing IDA with glyoxal in an 8:1 molar ratio gives varying amounts of the desired OXOEDTA. It appeared that heating the mixture above it's melting point gave a greater amount of OXOEDTA as well as the other component. It also appeared that more OXOEDTA was produced if the amount of water was low.

EXAMPLE 9: LARGE SCALE PREPARATION OF OXOEDTA AND HPLC CALIBRATION

In a flask containing 300 mL of acetonitrile and a magnetic stir bar, was dissolved 21.21 g (0.198 Mole) of benzyl amine. A 120g (0.868 Mole) portion of freshly ground (by mortar and pestle) anhydrous potassium carbonate was added, and the resulting suspension was stirred by mechanical stir-paddle. Tert-butyl-bromoacetate (77.22g, 0.396 Mole) was dissolved in 500 mL of acetonitrile, and the resulting solution was added over a 1 hour period to the benzylamine/potassium carbonate suspension. After 6.5 hours the suspension was vacuum filtered and the solid washed with 3-100 mL portions of acetonitrile. The combined filtrates were then concentrated at 40 °C in a rotary evaporator to remove the acetonitrile and give 67.55 g (102 percent yield) of di-tert-butyl (N-benzyl-3-aza)glutarate. A 66.18g (0.198 Mole) portion of di-tert-butyl (N-benzyl-3-aza)glutarate (hereinafter "the diester") was dissolved in 200 mL of 90 percent ethanol (made by adding 180 mL of 95 percent ethanol to 20 mL of water). A 1.0g portion of 10 percent palladium on carbon hydrogenation catalyst commercially available from Aldrich Chemical Company Inc. (catalog number 20,569-9) was mixed with 5 mL of 90 percent ethanol and transferred to the solution of the diester. The suspension was then exposed to a 50 p.s.i. (pounds per square inch) (345 KPa) atmosphere of hydrogen until 0.192 Mole of hydrogen was taken up (103 percent of theory). The suspension was then filtered to remove the hydrogenation catalyst taking care that the catalyst was kept wet with solvent to avoid fire. The filtrate was then concentrated under vacuum at 40 °C using a rotary evaporator to give 55.71g (114 percent yield) of di-tert-butyl (3-aza)glutarate.

A 125g (0.904 Mole) sample of freshly ground anhydrous potassium carbonate was added to a 3-necked 1L round-bottomed flask. A 48.71 g (0.170 Mole) portion of di-tert-butyl (3-aza)glutarate was dissolved in 400 mL of acetonitrile and added all at once to the potassium carbonate. Stirring of the suspension was achieved using a mechanical motor and stir-paddle. To the stirred suspension, was added a solution of 13.379 g (0.066 Mole) of bromoacetylbromide dissolved in 140 mL of acetonitrile. This suspension was then allowed to stir overnight (22 hours). The suspension was then vacuum filtered using 300 mL of acetonitrile to wash the solid. The filtrates were combined, and the solvent stripped under vacuum at 40 °C to give 42.08 g (93 percent) of oily material.

The oily material was dissolved in 62 mL of methylene chloride and purified on a 3 inch diameter by 21 inch tall silica gel chromatography column, with 2.5 percent methanol in methylene chloride used as eluent. The fractions containing the purified products were combined and the solvent removed under vacuum at 40 °C to give 19.68 g (56 percent yield) of the tetra-(tert-butyl)ester of OXOEDTA as a viscous clear oil which solidifies up on standing. The ester was identified by thin layer chromatography comparison with that of the ester isolated in Example 16.

A 17.33 g (32.7 mMole) portion of the tetra-(tert-butyl)ester of OXOEDTA was placed into a 500 mL round-bottomed flask and placed under a nitrogen atmosphere. To this oil, was added a stir bar and 100 mL of trifluoroacetic acid. After the solution was stirred for 2 hours, the trifluoroacetic acid was removed under vacuum at 40 °C, to produce a puffy white solid. This solid was dissolved in 100 mL of 3N HCl and then reduced to a volume of 30 mL under vacuum at 40 °C. The oil was then dissolved in an additional 30 mL of 3N HCl. Removal of the solvent at 40 °C under vacuum results in a crispy white solid. This solid was dissolved in 100 mL of water and passed through a 0.45 micron filter. The filtered solution was placed in a refrigerator overnight. The next day the pH of the solution was 2.90 and a precipitate had formed. The solid was vacuum filtered and washed with 3-10 mL portions of ice cold water to give 7.7904g (74 percent overall yield) of OXOEDTA as the monohydrate, melting point 150 °C (decomposes). (Elemental analysis for $C_{10}H_{14}N_2O_9$-$H_2O$: was calculated as C 37.04, H 4.97, N 8.64; and was found to be C 36.82, H 4.87, N 8.50) Thermogravimetric analysis of this sample of OXOEDTA on a DuPont 951 Thermal Gravimetric Analyzer coupled to a DuPont 2100 Thermal Analyzer Data Processing Unit with heating at 6 degrees/min. up to 120 °C and then 10 degrees/minute up to 320 °C indicated a 6.52 percent weight loss from 150 to 206.5 °C. This corresponded to loss of the hydrate water which would be a 5.56 percent weight loss.

A sample of the OXDEDTA was analyzed by proton and C-13 NMR at low pH of less than 1. The proton NMR spectrum has peaks at $\delta$ = 3.41 (2H), 3.18(4H), 3.05(2H), and 3.02(2H) from tetramethyl silane (TMS). The C-13 NMR spectrum has peaks at $\delta$ = 173.4, 173.1, 169.4, 165.2, 57.6, 57.2, 51.7 and 51.3 from TMS. Both were consistent with the expected structure wherein the acetate groups on the amide nitrogen are magnetically nonequivalent due to hindered rotation of the nitrogen-carbonyl bond.

HPLC CALIBRATION CURVE FOR OXOEDTA

An HPLC calibration curve for OXOEDTA was made using the OXOEDTA from this Example at different concentrations and recording the UV detector response. The calibration data is shown in Table 8:

Table 8

| HPLC CALIBRATION CURVE DATA FOR OXOEDTA | | | | | |
|---|---|---|---|---|---|
| Concentration of OXOEDTA (mM) | Retention time (mins) | UV Peak area ($X10^{-6}$) | Width at half-height (minutes) | Calculated UV Peak Area* | Ratio of area Found to Area Calculated |
| 10 | 13.05 | 151.68 | 1.68 | 152.35 | 99.56 |
| 5 | 13.57 | 79.09 | 1.72 | 77.34 | 102.27 |
| 2.5 | 13.74 | 39.22 | 1.73 | 39.84 | 98.45 |
| 1.25 | 14.42 | 20.62 | 1.81 | 21.1 | 97.76 |

*Least squares regression for UV area (y) versus concentration (x) gives the equation:
$y = mx + b$
where m = 15000748.19, and b = 2338612.87 with a correlation coefficient R = 0.9998. The concentrations (X) were then substituted into the equation to calculate the expected UV peak areas (y) shown in the Table.

EXAMPLE 10: PREPARATION OF OXOEDTA FROM IDA AND GLYOXAL AT DIFFERENT RATIOS

10A. An 8:1 molar ratio mixture of IDA to glyoxal was prepared by adding 11.17 g (63.1 mMole) of IDA to 1.1448 g (7.89 mMole) of 40 weight percent glyoxal in water, dissolving it in 25 mL of water and freeze-drying to give a yellow solid.

10A1. A 58 mg sample of A was weighed into a glass vial and put under nitrogen. The yellow solid was heated in an oil bath at 130° C for 2 minutes during which it foamed and turned dark. The resulting data solid was then dissolved in 7.4 mL of water to give a final pH of 10.36.

10A2. A 31 mg portion of A was weighed into a glass vial and put under nitrogen. The yellow solid was heated in an oil bath at 150° C for 2 minutes. The solid was then dissolved in 3.98 mL of water.

10B. A 4:1 molar ratio mixture of IDA to glyoxal was prepared by adding 5.581 g (31.58 mMole) of IDA to 1.1448 g (7.89 mMole) of 40 weight percent glyoxal in water, then dissolving it in 12.5 mL of water and freeze-drying to give a yellow solid with slightly less color than A above.

10B1. A 35.5 mg sample of B was weighed into a glass vial and put under nitrogen. The yellow solid was heated in an oil bath at 130° C for 2 minutes during which it foamed and turned more yellow than before heating. The solid was then dissolved in 9.3 mL of water to give a final pH of 10.08.

10B2. A 20.1 mg sample of B was weighed into a glass vial and put under nitrogen. The yellow solid was heated in an oil bath at 150° C for 2 minutes during which it foamed and turned more yellow than before heating. The solid was then dissolved in 5.25 mL of water.

10B3. A 24.3 mg sample of B was weighed into a glass vial and put under nitrogen. The yellow solid was heated in an oil bath at 150° C for 10 minutes during which it foamed and turned dark yellow. The solid was then dissolved in 6.4 mL of water to give a final pH of 10.04.

All of the above solutions were analyzed by HPLC, and the amount of OXOEDTA present was determined using the calibration curve described in Example 9. From the amount of OXOEDTA present, the percent of theoretical yield was calculated based on the limiting reagent, glyoxal.

Table 9

| HPLC ANALYSIS OF IDA + GLYOXAL REACTIONS | |
|---|---|
| Solution Analyzed (heating temp. and time | % Yield of OXOEDTA |
| Solution from 10A1 (130 °C for 2 minutes) | 8.9 |
| Solution from 10A2 (150 °C for 2 minutes) | 6.8 |
| Solution from 10B1 (130 °C for 2 minutes) | 15.4 |
| Solution from 10B2 (150 °C for 2 minutes) | 17.5 |
| Solution from 10B3 (150 °C for 2 minutes) | 17.1 |

These results indicate that lower ratios of IDA to glyoxal are preferred to get a higher yield of OXOEDTA. The results also indicate that at these lower ratios the higher temperature (150 °C) was preferred for short time periods.

EXAMPLE 11: AQUEOUS PREPARATION OF OXOEDTA FROM IDA AND GLYOXAL.

Into a 100 mL round bottomed flask was put 1.1448 g (7.89 mMole) of 40 weight percent glyoxal solution in water followed by 5.581 g (31.58 mMole) of IDA and 12 mL of water. The solution was heated to 70 °C with stirring. After 15 minutes, a 50 $\mu$L aliquot of the solution was removed and added to 4.866 mL of water and analyzed by HPLC to determine the UV area percent for OXOEDTA. After an additional 1 hour of heating, the reaction was again sampled. The results from these analyses are shown in Table 10:

Table 10

| ANALYSIS OF AQUEOUS REACTION OF IDA AND GLYOXAL | | | |
|---|---|---|---|
| Time of Sampling | UV Area Percent of IDA | UV Area Percent of Other Peak* | UV Area Percent of OXOEDTA |
| 15 minutes | 16 | 66 | 16 |
| 75 minutes | 16 | 67 | 16 |

*The largest peak appears between the IDA peak and the OXOEDTA peak and is as yet unidentified

These results show that OXOEDTA was produced under these conditions very early in the reaction but in a low amount that does not increase over time.

EXAMPLE 12: PREPARATION OF OXOEDTA FROM IDA AND ETHYL BROMOACETATE IN ETHYLENE GLYCOL

In a 50 mL glass vial, 2.076 g (9.38 mMole) of IDA disodium salt hydrate was dissolved in 40 mL of ethylene glycol. To this solution, was added 1/2 equivalent of ethyl bromoacetate. The resulting clear solution was allowed to stir for 63 hours after which it has not visibly changed. A 1 mL aliquot of the resulting solution was removed and heated at 150 °C for 50 minutes. After the solution was cooled to room temperature(25 °C), a precipitate was formed by addition of 10 mL of acetone to the solution. The solid was isolated by centrifuging and washing with acetone. The resulting yellow solid was then dissolved in 23 mL of water an analyzed by HPLC, with the results shown in Table 11:

Table 11

| HPLC ANALYSIS OF ISOLATED SOLID | | |
|---|---|---|
| Peak Identity | Retention time (min.) | UV Area Percent |
| IDA | 2.57 | 14.8 |
| ? | 4.12 | 2.3 |
| ? | 6.64 | 4.5 |
| OXOEDTA | 14.43 | 63.8 |
| ? | 23.6 | 14.5 |
| ? means unidentified product | | |

These results indicate that the major product (by UV area percent) in this reaction was OXOEDTA.

EXAMPLE 13: PREPARATION OF OXOEDTA BY SLOW ADDITION OF GLYOXAL TO IDA IN AQUEOUS CONDITIONS.

Into a 3-necked flask equipped with a heating mantle and reflux condenser, was added a solution of 10 g (75 mMole) of IDA acid in 30 mL of water. A 7.2g (90 mMole) portion of 50 weight percent NaOH was added to bring the pH up to 8.82. Then the solution was heated to 95 ° C under a nitrogen atmosphere. To the hot stirred solution, was added (via a dropping funnel) a solution of 1.72 mL (38 mMole) of 40 weight percent Glyoxal in 5 mL of nitrogen purged water. Immediately, a 238 mg aliquot of the resulting reaction mixture was removed and diluted into 26.98 mL of water. Likewise an aliquot was removed after 30 minutes at 95 ° C and at 22 hours at 95 ° C. Each aliquot was diluted with water. The UV area percent from the HPLC analyses of these aliquots was shown in Table 12:

Table 12

| UV AREA PERCENTS OF ALIQUOTS FROM THE AQUEOUS REACTION OF IDA WITH GLYOXAL | | | | |
|---|---|---|---|---|
| Time of Aliquot from the Reaction at 95 ° C | UV Area Percent for IDA | UV Area Percent for Other Peak(s)* | UV Area Percent for OXOEDTA | Calculated ** Percent Yield for OXOEDTA |
| 0 | 24 | 44 | 32 | 4.1 |
| 30 minutes | 22 | 39 | 38 | 5.2 |
| 22 hours | 22 | 41 | 38 | 5 |

*A peak intermediate in retention time between OXOEDTA and IDA was noted and was unidentified
**Based on glyoxal as the limiting reagent

EXAMPLE 14: SELECTIVE PRECIPITATION OF EDTA IN THE PRESENCE OF OXOEDTA

In a vial, a 171 mg (399 µMole) sample of OXOEDTA as prepared by the procedure of Example 20 was dissolved in 3.0 mL water. To this solution was added 171 mg (586 µMole) of EDTA. Not all of the EDTA was soluble (pH = 3.75), therefore 120 µL (975 uMole) of 25 weight percent NaOH was added such that the EDTA was dissolved. The final pH when all components were dissolved was 5.38, and the solution was 0.128 M in OXOEDTA and 0.188 M in EDTA.

The entire solution was treated with 600 µL (1.8 mMole) of 3N HCl solution which lowered the pH to 2.77. Some precipitate was noted immediately when the pH was lowered. The suspension was allowed to sit undisturbed for 24 hours and then was diluted by adding 3 mL of water and mixed vigorously to evenly distribute the powdery precipitate. A 50 µL aliquot of the resulting suspension was removed and dissolved in 1 mL of water by adding 10 µL of 25 weight percent NaOH after which it was analyzed by HPLC. The rest of the suspension was centrifuged, and a sample of the supernatent was analyzed by HPLC. The

centrifuged solid was washed with 3 mL of water and dried to give 150 mg (88 percent recovery) of EDTA. A 12.1 mg portion of this recovered EDTA was dissolved in 2 mL of water by the addition of 10 µL of 25 percent NaOH (final pH = 4) and analyzed by HPLC.

The UV areas for the peaks found in the HPLC analysis of EDTA and OXOEDTA for the whole suspension, the supernatent, and there covered EDTA precipitate were compared and are listed in Table 13:

These ratios show that the initial ratio of OXOEDTA peak area to EDTA peak area was about 10 even though the molar ratio of OXOEDTA to EDTA was 1:1.47. This was due to the

Table 13

| COMPARISON OF OXOEDTA/EDTA RATIOS IN DIFFERENT PHASES | | | | |
|---|---|---|---|---|
| Material Analyzed | UV Area of EDTA | UV Area for OXOEDTA | Ratio of UV Area for OXOEDTA to EDTA | Molar Ratio of EDTA to OXOEDTA |
| Whole suspension | 0.291 | 3.070 | 10.5 | 1.47:1 |
| Supernatent | 0.559 | 60.660 | 108.5 | 1:7 |
| Isolated Solid | 6.575 | 0.539 | 0.082 | 188:1 |

molar extinction coefficient of OXOEDTA being 15.5 times that of EDTA at the 210 nanometer wavelength used for this analysis. When the pH was lowered, a relatively pure form of EDTA was precipitated and the OXOEDTA/EDTA UV ratio was increased 10 fold in the supernatent (that is OXOEDTA was still soluble) and the molar ratio therein of OXOEDTA to EDTA was 7:1. 88 percent of the EDTA was recovered as a precipitate, leaving behind virtually all of the OXOEDTA. The EDTA to OXOEDTA molar ratio in the precipitated EDTA sample was 188:1 indicating less than 1 percent level of OXOEDTA present in the EDTA precipitate

These results taken together show that EDTA can be selectively precipitated in the presence of OXOEDTA and that OXOEDTA has much greater solubility at this low pH than does EDTA.

EXAMPLE 15 AND COMPARATIVE SAMPLE A: DISSOLUTION OF PIPE SCALE BY OXOEDTA

A cold water inlet pipe was removed from a home hot water heater and the pipe scale removed from the inner surface of the pipe. The lumpy deposits were ground to a fine reddish brown powder using a mortar and pestle.

An OXOEDTA solution was prepared in a plastic centrifuge tube containing 10 mL of water and 10 mL of 0.5 M sodium phosphate buffer (pH-7.3) by adding 203 mg (625 µMole) of OXOEDTA prepared by the procedure of Example 9. The tube was capped and shaken until all solids dissolve to give a solution 31.25 mM in OXOEDTA and 0.25 M in sodium phosphate at a pH of 6.38.

For Comparative Sample A, an EDTA solution was prepared in a plastic centrifuge tube containing 10 mL of water and 10 mL of 0.5 M sodium phosphate buffer (pH-7.3) by adding 182.6mg (625 µMole) of EDTA. The tube was capped and shaken until all solids dissolved to give a solution 31.25 mM in EDTA and 0.25M in sodium phosphate at a pH of 6.46.

For Comparative Sample B, an NTA solution was prepared in a plastic centrifuge tube containing 10 mL of water and 10 mL of 0.5 M sodium phosphate buffer (pH-7.3) by adding 119.5 mg (625 µMole) of NTA. The tube was capped and shaken until all solids dissolved to give a solution 31.25 mM in NTA and 0.25 M in sodium phosphate at a pH of 6.62

For Comparative Sample C, a water control was prepared in a plastic centrifuge tube by adding 10 mL of water and 10 mL of 0.5 M sodium phosphate buffer (pH-7.3).

Into each of the above 4 tubes, was placed 50.0 mg of the powder prepared from the deposits in the pipe. The tubes were capped and placed on an end over end rotator and were rotated at 20 rpm (revolutions per minute) at room temperature for 120 hours (5 days). The tubes were then centrifuged and the supernatent discarded. The remaining sediment was then washed with two 30 mL portions of water using centrifugation to isolate the sediment each time. The washed sediment was dried and weighed to determine how much of the original 50 mg was present. The data is shown in Table 14:

Table 14

| DISSOLUTION OF PIPE SCALE BY VARIOUS CHELANTS | | | | |
|---|---|---|---|---|
| Solution | pH (final) | Weight of Pipe Scale Left | % of Original 50 mg Left | % of Pipe Scale Dissolved |
| EXAMPLE 15 (OXOEDTA) | 6.46 | 0.0364g | 73 | 27 |
| COMPARATIVE SAMPLE A (EDTA) | 6.52 | 0.0365g | 73 | 27 |
| COMPARATIVE SAMPLE B (NTA) | 6.68 | 0.0348g | 70 | 30 |
| COMPARATIVE SAMPLE C (Water (Control)) | 7.14 | 0.0494g | 99 | 1 |

These results show that OXOEDTA, like other chelants such as EDTA and NTA can dissolve pipe scale.

Example 16: Synthesis and degradation of 2-oxo-1,4-ethylenediamine tetraacetic acid (OXOEDTA)

A. Preparation of di-tert-butyl (N-benzyl-3-aza) glutarate.

A flask was charged with 50 mL of acetonitrile containing 2.451 g (22.87 mMole) of benzylamine and 10 g (72.5) of freshly powdered potassium carbonate. The suspension was stirred using a magnetic stir bar and treated by adding (all at once) a solution of 8.920 g (45.7 mMole) of t-butyl bromoacetate dissolved in 10 mL of acetonitrile. After 18 hours of stirring at room temperature, the suspension was vacuum filtered and the filtrate evaporated under vacuum at 40°C to give 7.899 g (103 percent yield) of product as an oily semisolid. A 100 mg sample was dissolved in $CDC_{13}$ (deutero trichloromethane) for NMR (Nuclear Magnetic Resonance) analysis. The proton NMR shows a pattern consistent with di-tert-butyl-(N-benzyl-3-aza)glutarate and integration. (The spectrum had peaks at the following positions in ppm from an internal TMS standard: $\delta$ = 1.45 (singlet, t-butyl, 18H), 3.40 (singlet, $CH_2$,4H), 3.88 (singlet, benzyl $CH_2$,2H), 7.20-7.41 (multipet, phenyl CH,5H). The $C\text{-}_{13}$ NMR was also consistent with this structure and had peaks at 170.6, 138.5, 129.1, 128.3, 127.2, 80.8, 57.4, 55.1, 28.1 from an internal TMS standard.

B. Preparation of di-tert-butyl (3-aza) glutarate.

A 7.0 g (20.88mMole) portion of di-tert-butyl-(N-benzyl-3-aza)glutarate dissolved in 100 mL of 95 percent ethanol containing 0.5 g of 10 percent palladium on carbon catalyst was padded with nitrogen and exposed to hydrogen with shaking in a Parr hydrogenation laboratory apparatus set up according to the method described in Bergeron and Garlich, Synthesis page 782, 1984, until hydrogen uptake ceases. The catalyst was then filtered and washed with alcohol taking care that the catalyst was kept wet to avoid fire. The solvent was then stripped from the filtrate under vacuum at 40°C. The resulting oil was dissolved in 50 mL of CHCl3 (trichloromethane) dried over sodium sulfate, decanted, and the supernatent was reconcentrated under vacuum to give 4.99 g (97 percent yield) of desired product as a thick oil. A 100 mg sample was dissolved in 1 mL of $CDCl_3$ for NMR analysis (proton and C-13) which was consistent with the proposed structure. The proton NMR has peaks at $\delta$ = 1.42 (singlet, t-butyl, 18H), 2.18 (singlet, NH, 1H), 3.28 (singlet, $CH_2$, 4H) from TMS. The C-13 NMR has peaks at $\delta$ = 170.9, 81.2, 50.8 and 28.0 from TMS.

C. Preparation of the tetra-(tert-butyl) ester of OXOEDTA.

For this preparation, glassware was dried in an oven at 140°C for 1 hour and allowed to cool to 60°C before use to minimize the presence of the water to which this reaction was sensitive. A 100mL flask equipped with a large magnetic stir bar was charged with 25 mL of acetonitrile (containing less than 0.002 percent water) and 4.0 g (29 mMole) of freshly ground potassium carbonate. To this stirred suspension, was added 10 mL of acetonitrile containing 3.340 g (13.63 mMole) of di-tert-butyl (3-aza) glutarate and then 1.070 g (6.815 mMole) of commercially available bromoacetylbromide. The suspension was allowed to stir under a nitrogen pad overnight at room temperature. After 16 hours, the suspension was vacuum filtered and the filtrate concentrated under vacuum at 40°C to give 3.484 g (94 percent yield) of crude product. The product was purified by silica gel comumn chromatography, with 2.5 weight percent MeOH in $CH_2C1_2$ used as eluent. The fractions containing the product as determined by thin layer chromatography were combined and evaporated under vacuum at 40°C to give 2.7165 g (75 percent yield) of the tetra-(tert-butyl)ester of

OXOEDTA. A 110 mg sample of this ester was dissolved in 1 mL of $CDCl_3$ for NMR (proton and C-13) analysis which was consistent with the proposed structure as indicated by peaks at $\delta$ = 1.41-1.44 (multiplet, t-butyl, 36H), 3.40 (singlet, $CH_2$, 4H), 3.62 (singlet, $CH_2$, 2H), 3.97 (singlet, $CH_2$, 2H), 4.53 (singlet, $CH_2$, 2H) from TMS in the proton NMR and at $\delta$ = 170.2, 169.9, 169.0, 168.2, 81.7, 81.0, 55.8, 55.5, 50.6, 49.1, 28.1 from TMS in the C-13 NMR.

D. Preparation of OXOEDTA.

Into a 500mL flask equipped with a large magnetic stir bar, was placed 2.6065 g (4.918 mMole) of the tetra-(tert-butyl)ester of OXOEDTA and 15 mL of commercially available trifluoroacetic acid. After the resulting solution was stirred for 3 hours, the stir bar was rinsed with 3 mL of trifluoroacetic acid and removed. The remaining solution was concentrated under vacuum at 40 °C to give a puffy white solid. The solid was dissolved in 15 mL of 3N (Normal) HCl (hydrochloric acid) and again concentrated under vacuum at 40 °C to a white solid. This procedure was repeated again and the resulting white solid dissolved in 15 mL of water and concentrated under vacuum to dryness. The resulting solid was dissolved in 25 mL of water and lyophilized to give 1.4978 g (105 percent yield including impurities) of white hygroscopic solid. The OXOEDTA was analyzed by proton and C-13 NMR (in $D_2O$) which were consistent with the proposed structure as indicated by peaks at $\delta$ = 4.04 (Singlet, $CH_2$, 2H), 4.06 (Singlet, $CH_2$, 2H), 4.11 (Singlet, $CH_2$, 4H), 4.38 (Singlet, $CH_2$, 4H) from TMS in the proton NMR and $\delta$ = 174.7, 174.4, 170.9, 169.4, 57.9 (2C), 52.3, 51.9 from TMS in the C-13 NMR.

E. Photodegradation of OXOEDTA

A 0.01M solution of OXOEDTA was prepared by dissolving 122 mg of solid isolated in "D" above in 20 mL of water, adding 2N NaOH, and then diluting to 40 mL with water. The resulting solution was 0.01M in OXOEDTA with a pH of 5.68. A 2 mL portion of this solution was placed into a standard quartz UV (ultraviolet) cell and placed in front of a 100-watt Hanovia UV lamp commercially available from Ace Glass Incorporated (4 cm away). Aliquots were taken at time intervals and analyzed by HPLC (High Pressure Liquid Chromatography) using an anion exchange column, commercially available from Hamilton Company under the trade designation Hamilton PRP-X100 (4.1mmX25cm column), eluting with a solution of 8 volume percent 0.1N $H_2SO_4$ and 92 volume percent water at 1 mL per minute, and using UV detection at 210nm. The area of the OXOEDTA peak from these chromatograms in this UV degradation versus time study are shown in Table 15.

Table 15

| Time | Area of OXOEDTA Signal | Percent OXOEDTA remaining |
|---|---|---|
| 0 | 22.102 | 0 |
| 10 minutes | 19.133 | 86.6 |
| 1 hour | 16.220 | 73.4 |
| 2.5 hours | 10.084 | 45.6 |
| 4.5 hours | 4.079 | 18.5 |
| 19.5 hours | 0 | 0 |

This data shows that OXOEDTA was degraded by UV light within 19.5 hours at the intensity used.

EXAMPLE 17: Complexation of OXOEDTA with Fe(III).

A 5 mL portion of 0.01M OXOEDTA prepared as in Example 16 was added to 2.5 mL of 0.01M $FeCl_3$ with stirring. The pH of the solution was then adjusted from 3.06 to 12.01 by adding 0.1N NaOH (sodium hydroxide) in 100-300 $\mu$L portions. At this pH, all components were in solution, but after the solution sat overnight, a reddish orange precipitate formed leaving a water white supernatenant.

A 5 mL portion of 0.01 M EDTA mixed with an FeCl3 solution in the same manner also formed a reddish orange precipitate overnight and left a water white supernatent.

This Example shows that OXOEDTA, like EDTA, can complex iron and keep it solubilized even at high pH for a significant time period.

EXAMPLE 18 AND COMPARATIVE SAMPLE D: Photodegradation of the Iron Complex of OXOEDTA.

For Example 18, a solution of the OXOEDTA complex with iron (FOE) was prepared by adding 2.5 mL of 0.01M OXOEDTA as prepared in Example 16 to 1.25 mL of 0.01 M $FeCl_3$ solution and adjusting the pH to 11.38 by the addition of 100mL of 1N NaOH. The pH of the solution was quickly adjusted down by adding 66 $\mu$L of 3N HCl to give a final pH of 1.92.

For Comparative Sample D, a complex of EDTA with iron (FE) was made by the same procedure: a 2.5 mL portion of 0.01M EDTA was added to 1.25 mL of 0.01M $FeCl_3$, the pH adjusted to 11.25 by adding 100 $\mu$L of1N NaOH, then the pH adjusted to 11.25 by adding 100 $\mu$L of 1N NaOH, then the pH adjusted to 1.93 by the addition of 54 $\mu$L of 3N HCl.

Both the complex containing solutions (FE and FOE complexes) were heated at 77°C for 17 hours and allowed to cool to room temperature. The FE solution C.S. D.) was yellow with no precipitate, and the FOE solution (Ex. 18) was yellow with a faint trace of yellow precipitate. The FOE solution at a pH of 2.03 was treated with 1.2 mL of 0.1 N NaOH in 100 $\mu$L portions to give a final clear yellow solution of the complex at a pH of 8.05. The FE solution at a pH of 1.98 was treated with 1.25 mL of 0.1N NaOH in 100 $\mu$L portions to give a final water white solution of the complex at a pH of 8.28.

A 2 mL aliquot of each complex solution was put into a separate quartz UV cuvette and placed within 4 cm of a water jacketed 100 watt Hanovia UV lamp as described in Example 16E. After 3.5 hours of UV exposure, the samples were removed from the light source. The FE solution (C.S. D) was yellow with faint turbidity. The FOE solution (Ex. 18) was yellow with a dark yellow solid in the bottom.

A 1-mL aliquot of each irradiated sample (after shaking to ensure homogeneity) as well as a 1 mL aliquot of each complex solution that had not been irradiated was centrifuged for 15 minutes at full speed on a Clay-Adams tabletop centrifuge commercially available from Becton, Dickerson and Company under the trade designation Safety Head Centrifuge. A 500 $\mu$L portion of the supernatent from each sample was analyzed by inductively coupled plasma spectrometry using an inductively coupled plasma atomic emmission spectrometer commercially available from Perkin Elmer Corporation under the trade designation Model 6500 according to manufacturer's directions. Results of the analyses show:

For Example 18:
Iron Concentration in FOE Solution Before Irradiation = 5.432ppm
Iron Concentration in FOE Solution After Irradiation = 2.264ppm
Percent Reduction in Soluble Iron Due to Irradiation = 58 percent
For Comparative Sample D:
Iron Concentration in FE Solution Before Irradiation = 6.198ppm
Iron Concentration in FE Solution After Irradiation = 5.538ppm
Percent Reduction in Soluble Iron Due to Irradiation = 11 percent

These data show that the iron complex of OXOEDTA was more susceptible to ultraviolet degradation than was the corresponding iron complex of EDTA.

Example 19: BIODEGRADABILITY SCREENING VIA ASTM D2667 SEMI-CONTINUOUS ACTIVATED SLUDGE TEST

The procedure of ASTM D-2665-82 was used to determine the inherent biodegradablity of OXOEDTA.

Copper titration value is used to measure the extent of biodegradation of the chelating agents during the procedure. Titration was performed using ammonium purpurate (indicator for complexometric titration commercially available from Aldrich Chemical Co., Inc. under the trade designation Murexide) as the indicator at approximately pH 8, and using sodium acetate as buffer. Titration of 6.12 mg OXOEDTA (0.02 mMoles) in 100 ml water with 0.01 molar copper chloride gave an end point of 1.95 ml (0.02 mMoles), representing a 1:1 chelation of copper. Analyses were performed daily for a period of 28 days.

Results of the biodegradation screening:

OXOEDTA showed 80 percent loss of chelation capacity (indicating degradation) within 26 days while NTA showed greater than 80 percent degradation within 8 days.

No measurable loss of EDTA was noted within the 28 day test period. Extending the test to 52 days, still resulted in no measureable loss of EDTA.

A control was used to verify the abscence of interfering chelating substances in the test.

These results of the biodegradability test show that OXOEDTA material is inherently biodegradable and

could be expected to be utilized by organisms in a municipal treatment facility after an acceptable acclimation period.

Example 20: PREPARATION OF CRUDE OXOEDTA

A 40 g portion of freshly ground potassium carbonate was added to 9.803 g (91.48 mMole) of benzyl amine dissolved in 250 mL of acetonitrile. With stirring, the suspension was treated with 35.656 g (0.183 Mole) of t-butyl bromoacetate dissolved in 50 mL of acetonitrile. A pan of water was placed under the reaction to keep the reaction temperature below 30 ° C. The suspension was stirred overnight after which TLC (Thin Layer Chromatography) indicated that all benzyl amine had been consumed (silica gel TLC developed in 10 percent $MEOH/CHCl_3$). The suspension was vacuum filtered, and the solid was washed with three 42 mL portions of acetonitrile. The combined filtrates were then rotary evaporated at 40 ° C to give 35.98 (117 percent yield) of yellow tinted oil which solidified upon standing. A 35.15 g portion of this compound was then dissolved in 200 mL of 95 weight percent ethanol and treated with 1.0 g of 10 percent paladium on carbon (Pd/C) and hydrogen gas to remove the benzyl protecting group. After hydrogen uptake ceases (99.7 percent of theory), the catalyst was removed by vacuum filtration and the ethanol was removed by rotary evaporation at 40 ° C. This results in 24.682 g (113 percent yield) of di-tert-butyl (3-aza)-glutarate.

All of the di-tert-butyl (3-aza)glutarate was dissolved in 200 mL of acetonitrile, stirred with 50 g of freshly ground potassium carbonate and treated with 6.839 g (33.9 mMole) of bromoacetyl bromide dissolved in 70 mL of acetonitrile in 20 mL portions over a 3 minute period. After 15 hours of stirring the suspension was vacuum filtered and concentrated under vacuum at 40 ° C to give 19.80 g of the tetra(t-butyl ester of OXOEDTA as a pale yellow viscous oil.

A 17.91 g portion of the tetra(t-butyl)ester of OXOEDTA was treated with 25 mL of trifluoroacetic acid (TFA) for 4 hours after which the TFA was removed under vacuum to produce a puffy solid which was then taken up in 100 mL of 3N HCl and rotary evaporated at 40 ° C under vacuum. This was repeated to give 12.91 of solid crude OXOEDTA. HPLC (High Pressure Liquid Chromatography) analysis of this material shows 91 percent of the peak areas attributable to OXOEDTA. All of this crude OXOEDTA was dissolved in 100 mL of water and treated with 12.43 g of 50 weight percent NaOH (0.155 Moles of Na + ) to give a solution of the sodium salt of OXOEDTA (hereinafter NaOXOEDTA) at a pH of 7.83. This solution of NaOXOEDTA was freeze-dried to give 17.77 g of solid NaOXOEDTA. 70 mL of acetonitrile in 20 mL portions over a 3 minute period. After 15 hours of stirring the suspension was vacuum filtered and concentrated under vacuum at 40 ° C to give 19.80 g of the tetra(t-butyl ester of OXOEDTA as a pale yellow viscous oil.

A 17.91 g portion of the tetra(t-butyl)ester of OXOEDTA was treated with 25 mL of trifluoroacetic acid (TFA) for 4 hours after which the TFA was removed under vacuum to produce a puffy solid which was then taken up in 100 mL of 3N HCl and rotary evaporated at 40 ° C under vacuum. This was repeated to give 12.91 of solid crude OXOEDTA. HPLC (High Pressure Liquid Chromatography) analysis of this material shows 91 percent of the peak areas attributable to OXOEDTA. All of this crude OXOEDTA was dissolved in 100 mL of water and treated with 12.43 g of 50 weight percent NaOH (0.155 Moles of Na + ) to give a solution of the sodium salt of OXOEDTA (hereinafter NaOXOEDTA) at a pH of 7.83. This solution of NaOXOEDTA was freeze-dried to give 17.77 g of solid NaOXOEDTA.

**Claims**

**1.** A compound represented by Formula 1:

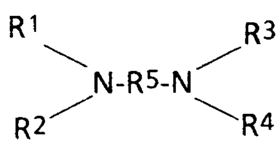

wherein each of $R^1$, $R^2$, $R^3$, and $R^4$ is independently H, hydroxyalkyl, $-C(R^6)_2COOH$, or ammonium, amine, or alkali metal salts thereof, but at least 3 of $R^1$, $R^2$, $R^3$, and $R^4$ are $-C(R^6)_2COOH$ or salts thereof; $R^5$ is an alkylene group having at least one carbonyl group; and each of $R^6$ is independently selected from H or a straight or branched chain alkyl group of from 1 to 4 carbon atoms.

2. The compound of Claim 1 wherein one of $R^1$, $R^2$, $R^3$, or $R^4$ is hydroxyalkyl of from 1 to 5 carbon atoms and at least 2 of $R^1$, $R^2$, $R^3$ and $R^4$ are $-C(R^6)_2COOH$ or salts thereof.

3. The compound of Claims 1 or 2 wherein all of $R^1$, $R^2$, $R^3$ and $R^4$ are - $C(R^6)_2COOH$ or salts thereof, and $R^5$ has from 2 to 3 carbon atoms and one carbonyl group.

4. The compound of Claims 1, 2, or 3 wherein all of $R^1$, $R^2$, $R^3$ and $R^4$ are - $C(R^6)_2COOH$ or salts thereof and $R^5$ is

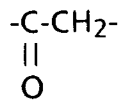

5. The compound of Claims 1,2, 3, or 4 wherein each of $R^6$ is H which is N,N'-(1-oxo-1,2-ethanediyl)-bis-[N-(carboxymethyl)-glycine].

6. The compound of Claims 1, 2, 3, 4 or 5 wherein each of $R^6$ is H.

7. A washing composition comprising an organic detergent surfactant selected from the group consisting of anionic detergents, cationic detergent, nonionic detergents, ampholytic detergents, zwitterionic detergents, and mixtures of such detergents suitable for use in water and at least one water-soluble salt of the acids of Formula 1 selected from the group consisting of alkali metal salts, ammonium salts, and alkyl ammonium salts.

8. The composition of Claim 7 wherein the weight ratio of detergent surfactant to the salt is from 100:1 to 3:2; said water-soluble salt is a sodium salt; and said detergent surfactant is one or a mixture of anionic detergents.

9. The composition of Claim 7 or 8 additionally containing from 2 to 10 percent by weight based on the total weight of the composition of a water-soluble alkali metal silicate; or up to 60 percent by weight based on the total weight of the composition of an alkali metal sulfate or an alkali metal carbonate, or both.

10. An aqueous washing system consisting essentially of water, an organic detergent surfactant selected from the group consisting of anionic detergents, cationic detergents, nonionic detergents, ampholytic detergents, zwitterionic detergents, and mixtures of such detergents and at least one water-soluble salt of an acid of Formula 1 selected from the group consisting of alkali metal salts, ammonium salts, and alkyl ammonium salts, the ratio by weight of the detergent surfactant to the salt being in the range of from 100:1 to 3:2, said system having a pH between 8 and 12.

11. The method of washing articles which comprises contacting the same with an aqueous washing system of Claim 10.

12. A process of removing $H_2S$ from a fluid comprising contacting said fluid with an aqueous solution at a pH suitable for removing $H_2S$ wherein said solution contains at least one higher valence polyvalent metal chelate of Formula 1.

13. A process of removing $NO_x$ from a fluid comprising contacting the fluid with an aqueous solution of at least one lower valence state polyvalent metal chelate of Formula 1 or 2.

14. A method of chelating a metal ion comprising contacting the metal ion with at least one compound of Formula 1.